# FASCICULE DE BREVET EUROPEEN

(11) **EP 1 093 799 B1**
(45) Date de publication et mention de la délivrance du brevet: **07.08.2002**
(21) Numéro de dépôt: 00402819.7
(22) Date de dépôt: 12.10.2000
(51) Int. Cl.: A61K 7/42, A61K 7/40, A61K 7/02, A61K 7/06

(54) **Emulsions eau-dans-huile contenant au moins un filtre UV organique insoluble et une silicone organomodifiée non-filtrante**
Wasser-in-Öl Emulsionen enthaltend mindestens ein unlössliches organisches UV Filter und ein nicht-filtrierendes organomodifiziertes Silikon
Water-in-Oil emulsions containing at least one insoluble organic UV filter and a non-filtrating organomodified silicone

(30) Priorité: 22.10.1999 FR 9913219
(43) Date de publication de la demande: 25.04.2001
(73) Titulaire: L'OREAL, 75008 Paris (FR)
(72) Inventeur: Candau, Didier, 91570 Bievres (FR); Forestier, Serge, 77410 Claye Souilly (FR)
(74) Mandataire: Miszputen, Laurent

(56) Documents cités:
- FR-A- 2 771 926
- GB-A- 2 286 774

## Description

La présente invention a pour objet une émulsion eau-dans-huile cosmétique ou dermatologique, caractérisée par le fait qu'elle comporte :
- au moins une phase aqueuse et
- au moins une phase grasse ;
- au moins un système photoprotecteur capable de filtrer les rayons UV contenant au moins un filtre UV organique insoluble dans ladite émulsion, sous forme micronisée ;
- au moins une silicone organomodifiée non-filtrante.

L'invention concerne également ses utilisations pour la fabrication de compositions cosmétiques ou dermatologiques pour la photoprotection de la peau ou des cheveux.

L'invention concerne également ses utilisations pour la fabrication de compositions cosmétiques ou dermatologiques pour la photoprotection de la peau ou des cheveux.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photoallergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion, de type huile-dans-eau (c'est à dire un support cosmétiquement et/ou dermatologiquement acceptable constitué d'une phase continue dispersante aqueuse et d'une phase discontinue dispersée grasse) ou eau-dans-huile (phase aqueuse dispersée dans une phase grasse continue), qui contient, à des concentrations diverses, un ou plusieurs filtres organiques classiques, lipophiles et/ou et/ou des nanopigments minéraux d'oxydes métalliques, capables d'absorber sélectivement les rayonnements UV nocifs, ces filtres (et leurs quantités) étant sélectionnés en fonction du facteur de protection solaire recherché (le facteur de protection solaire (SPF) s'exprimant mathématiquement par le rapport du temps d'irradiation nécessaire pour atteindre le seuil érythémal avec le filtre UV au temps nécessaire pour atteindre le seuil érythémal sans filtre UV). Dans de telles émulsions, les filtres hydrophiles sont présents dans la phase aqueuse et les filtres lipophiles sont présents dans la phase grasse.

Les émulsions huile-dans-eau sont, d'une manière générale, plus appréciées par le consommateur que les émulsions eau-dans-huile, en raison notamment de leur toucher agréable (voisin de l'eau) et de leur présentation sous forme de lait ou de crème non gras; cependant, elles perdent également plus facilement leur efficacité en protection UV dès lors qu'elles viennent en contact avec l'eau; en effet, les filtres hydrophiles, ont tendance à disparaître à l'eau, par baignade en mer ou en piscine, sous la douche ou lors de la pratique de sports nautiques ; ainsi, les compositions solaires qui les contiennent, seuls ou associés aux filtres lipophiles, n'apportent plus la protection initiale recherchée dès lors que le substrat (peau ou cheveu) sur lequel elles ont été appliquées vient en contact avec l'eau.

On peut disposer de compositions antisolaires présentant une résistance à l'eau améliorée en mettant en oeuvre des émulsions eau-dans-huile. En effet, un filtre hydrophile est plus rémanent à l'eau au sein d'une émulsion eau-dans-huile qu'au sein d'une émulsion huile-dans-eau. Cependant, comme il a été indiqué plus haut, de telles compositions ne donnent pas encore entièrement satisfaction dans la mesure où elles laissent après application une impression de gras particulièrement désagréable pour l'utilisateur.

Ainsi, le besoin subsiste toujours quant à pouvoir disposer de compositions antisolaires apportant à la peau et/ou aux cheveux une protection solaire efficace, stable dans le temps et résistante à l'eau (rémanence à l'eau) et dont les performances cosmétiques seraient comparables à celles obtenues avec les émulsions huile/eau classiques.

La Demanderesse a découvert de manière surprenante et inattendue que des émulsions particulières contenant au moins un filtre UV organique insoluble dans les phases de l'émulsion, sous forme insoluble micronisée et au moins une silicone organomodifiée particulière non-filtrante permettaient non seulement d'obtenir des compositions antisolaires dont les performances cosmétiques étaient comparables à celles obtenues généralement avec une composition antisolaire classique sous forme d'émulsion huile/eau ou eau/huile mais aussi présentaient une bonne stabilité ainsi qu'une rémanence à l'eau améliorée.

Ces découvertes sont à l'origine de la présente invention.

La présente invention a pour objet une émulsion eau-dans-huile cosmétique ou dermatologique, caractérisée par le fait qu'elle comporte :
(a) au moins une phase aqueuse et
(b) au moins une phase grasse;
(c) au moins un système photoprotecteur capable de filtrer les rayons UV contenant au moins un filtre UV organique insoluble dans ladite l'émulsion, sous forme micronisée dont la taille moyenne des particules varie de 0,01 à 2µm ;
(d) au moins une silicone organomodifiée non-filtrante comportant des groupements oxyalkylénés ;
sous réserve que ledit filtre UV organique soit différent de la 2,4,6-tris[p-(2'-éthylhexyl-1'-oxycarbonyl)-anilino]-1,3,5-triazine sous forme insoluble micronisée et du composé de structure : 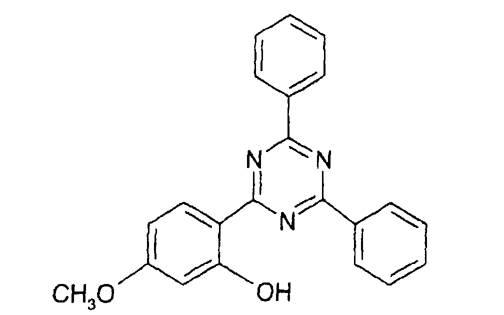 sous forme insoluble micronisée.

Par émulsion cosmétique ou dermatologique, au sens de la présente invention et dans le texte qui suit, on entend toute émulsion dont la phase aqueuse et la phase grasse sont constituées de substances cosmétiquement ou dermatologiquement acceptables pour une application topique sur les matières kératiniques humaines incluant la peau, les cheveux, les cils, les sourcils, les lèvres, les ongles ou les muqueuses.

Par silicone non-filtrante, au sens de la présente invention et dans le texte qui suit, on entend toute silicone ne comportant pas dans sa structure de groupe fonctionnel absorbant les radiations UV.

Par filtre UV organique insoluble, on entend, au sens de la présente invention, des filtres UV organiques insolubles dans les milieux cosmétiques généralement utilisés dans les formulations solaires et plus particulièrement dont la solubilité dans l'eau à 25°C est inférieure à 0,1 % en poids et dont la solubilité dans l'huile de paraffine à 25°C est inférieure à 1% en poids.

Par système photoprotecteur capable de filtrer les rayons UV, on entend par tout système constitué d'un ou plusieurs composés organiques et/ou composés minéraux filtrant les radiations UVA et/ou UV-B.

La présente invention a également pour objet l'utilisation de l'émulsion pour la fabrication de compositions cosmétiques pour la protection de la peau et/ou des cheveux contre le rayonnement ultraviolet, en particulier le rayonnement solaire.

La présente invention a également pour objet l'utilisation d'une silicone organomodifiée non-filtrante comportant des groupements oxyalkylénés pour la fabrication d'une émulsion cosmétique ou dermatologique photoprotectrice contenant au moins filtre UV organique insoluble dans ladite émulsion, sous forme micronisée dont la taille moyenne des particules varie de 0,01 à 2µm dans le but d'augmenter la résistance à l'eau de son pouvoir filtrant (rémanence à l'eau).

D'autres caractéristiques, aspects et avantages de la présente invention apparaîtront à la lecture de la description détaillée qui va suivre.

On entend par silicone, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁-C₁₀ et en particulier méthyle, les radicaux fluoroalkyles, les radicaux aryles et en particulier phényle. Les silicones sont définies plus en détail dans l'ouvrage de Walter NOLL "Chemistry and Technology of Silicones" (1968) Academie Press.

Les silicones organomodifiées utilisables conformément à l'invention sont des silicones telles que définies précédemment et comportant dans leur structure un ou plusieurs groupements organofonctionnels fixés par l'intermédiaire d'un radical hydrocarboné. Les silicones utilisables conformément à l'invention peuvent se présenter sous forme d'huiles, de cires, de résines ou de gommes. Elles peuvent être hydrosolubles ou insolubles dans l'eau.

Parmi les silicones non filtrantes comportant des groupements oxyalkylénés (en particulier oxyéthylénés et/ou oxypropylénés) on utilisera plus particulièrement les composés répondant à la formule suivante : 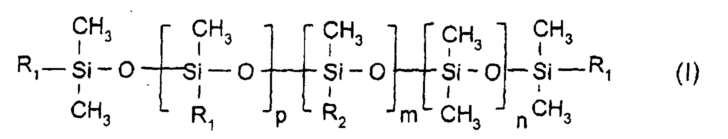
- R₁, identique ou différent, représente un radical alkyle, linéaire ou ramifié, en C₁-C₃₀ ou phényle.
- R₂, identique ou différent, -C_{c}H_{2c}-(-O-C₂H₄)ₐ-(-O-C₃H₆)_{b}-(O-C₄H₈)_{d}-R₃,
- R₃, identique ou différent, est choisi parmi un atome d'hydrogène, un radical hydroxyle, un radical alkyle linéaire ou ramifié de 1 à 12 atomes de carbone, un radical alcoxy linéaire ou ramifié de 1 à 6 atomes de carbone, un radical acyloxy linéaire ou ramifié de 2 à 12 atomes de carbone, NHCH₂CH₂COOM, aminoalkyle éventuellement substitué sur l'amine, carboxyacyle en C₁-C₃₀, un groupement phosphono éventuellement substitué, -O-CO-(CH₂)_{d}-CO₂M, -NHCO(CH₂)_{d}OH, -NH₃Y.
- M, identique ou différent, désigne un atome d'hydrogène, Na, K, Li, NH₄ ou une amine organique, Y représente un anion minéral ou organique monovalent tel que halogénure (chlorure, bromure), sulfate, carboxylate (acétate, lactate, citrate).
- a varie de 0 à 100,
- b varie de 0 à 50,
- c varie de 0 à 5,
- a + b est supérieur ou égal à 1,
- d varie de 0 à 10,
- m varie de 0 à 20,
- n varie de 0 à 500,
- p varie de 0 à 50,

On peut citer par exemple les composés vendus sous les dénominations commerciales FLUID DC 193 par la société DOW CORNING, SILWET L 77 par la société OSI et MAZIL 756 par la société MAZER PPG ; on peut citer également les produits commercialisés sous les noms « Silicone DC 3225C » « DC Q2-5200 » par la société Dow Corning .

Dans la définition ci-dessus et dans la suite du texte, OE représente une mole d'oxyde d'éthylène et OP représente une mole d'oxyde de propylène.

En particulier, une première famille de silicones non filtrantes comportant des groupements oxyalkylénés convenant particulièrement bien aux compositions selon l'invention est celle des composés répondant à la formule (I) ci-dessus pour lesquels les radicaux R₁ représentent tous des radicaux méthyle et le radical R₃ représente un radical hydroxy.

A titre d'exemple d'émulsionnant siliconé appartenant à cette famille, on peut citer le poly diméthyl / méthyl siloxane oxyéthyléné oxypropyléné (OE/OP 18/18) pour lequel p+n est 396 et m est 4, (nom CTFA : Cyclométhicone 90% Dimethicone copolyol 10%) vendu sous la dénomination commerciale de « Silicone Q2-3225C » par la société Dow Corning.

Une deuxième famille de silicones non filtrantes comportant des groupements oxyalkylénés convenant particulièrement bien aux compositions selon l'invention est celle des composés répondant à la formule (I) ci-dessus pour lesquels les radicaux R₁ représentent des radicaux méthyle et des radicaux lauryle et le radical R₃ représente un radical hydroxy.

Un émulsionnant siliconé particulièrement préféré de cette deuxième famille est le poly méthyllauryl / méthyl siloxane oxyéthyléné oxypropyléné (OE/OP 18/18) pour lequel p+n est 35 et m est 3, (nom CTFA : Laurylmethicone copolyol 91%, Isostearyl alcohol 9%) vendu sous la dénomination commerciale « DC Q2-5200 » par la société Dow Corning.

Un autre émulsionnant siliconé particulièrement préféré de cette deuxième famille est le poly méthylcetyl / méthyl siloxane oxyéthyléné oxypropyléné, pour lequel p est 20 à 25 et m est 5 et n est 75 (nom CTFA : cetyldimethicone copolyol) vendu sous la dénomination commerciale « Abil EM 90 » par la société Goldschmidt.

Un émulsionnant siliconé tout particulièrement préféré pour les compositions selon l'invention est une silicone oxyalkylénée substituée en α-ω à structure linéaire, substituée aux deux extrémités de la chaîne principale par des groupements oxyalkylène reliés aux atomes de Si par l'intermédiaire d'un groupement hydrocarboné. On choisira plus particulièrement une silicone répondant à la formule générale (II) suivante : 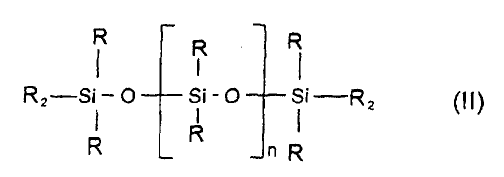 dans laquelle n varie de 1 à 500 ; les radicaux R₂, identiques ou différents, désignent un groupe -C_{c}H_{2c}-(-O-C₂H₄)ₐ-(-O-C₃H₆)_{b}-(O-C₄H₈)_{d}-R₃ où a, b, c et R₃ ont les mêmes définitions indiquées dans la formule (I) ci-dessus et les radicaux R représentent, identiques ou différents un radical alkyle, linéaire ou ramifié en C₁-C₃₀ ou phényle.

Et plus préférentiellement, tous les radicaux R sont des radicaux méthyles ; c va de 2 à 4, a va de 3 à 100, b va de 1 à 50, d vaut 0, R₃ est un radical hydroxy , n va de 50 à 200.

De préférence, le rapport en poids des unités C₂H₄O par rapport aux unités C₃H₆O va de 100:10 à 20:80. De façon avantageuse, ce rapport est d'environ 58/42.

Parmi les produits du commerce pouvant contenir tout ou partie des silicones oxyalkylénées substituées en α-ω utilisables selon l'invention comme émulsionnant, on peut citer notamment ceux vendus sous les dénominations de "Abil EM 97" par la Société Goldschmidt, ou encore de "KF 6009", "X22-4350", "X22-4349" ou "KF 6008" par la Société Shin Etsu.

La ou les silicones organomodifiées non filtrantes selon la présente invention sont de préférence présentes dans des concentrations allant de 0,1 à 20 % par rapport au poids total de la composition, et plus préférentiellement en une quantité allant de 0,5 à 10 %.

Les filtres UV organiques insolubles sous forme micronisée conformes à l'invention peuvent être choisis notamment parmi les filtres UV organiques du type oxanilide, du type triazine, du type triazole, du type amide vinylique, du type cinnamide.

Parmi les filtres UV du type oxanilide conformes à l'invention, on peut citer ceux répondant à la structure : 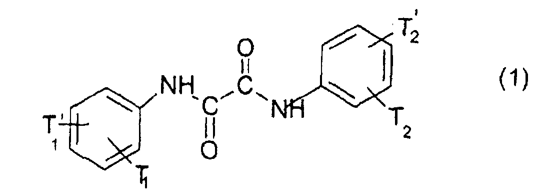 dans laquelle T₁, T'₁, T₂ et T'₂ désignent, identiques et différents, un radical alkyle en C₁-C₈ ou un radical alcoxy en C₁-C₈. Ces composés sont décrits dans la demande de brevet WO95/22959. A titre d'exemples, on peut citer les produits commerciaux TINUVIN 315 et TINUVIN 312 vendus par la Société CIBA-GEIGY et respectivement de structure :

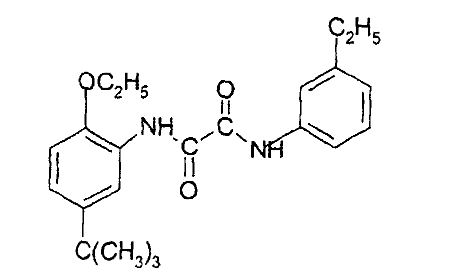

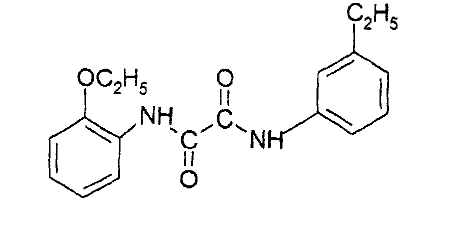

Les dérivés de 1,3,5-triazine conformes à l'invention préférentiels répondent à la formule générale suivante : 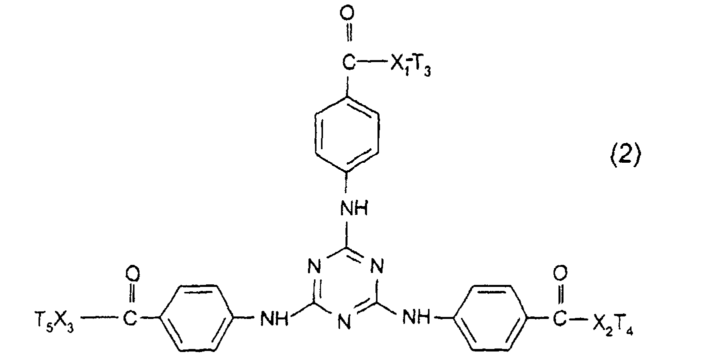 dans laquelle :
- X₁, X₂ et X₃, identiques ou différents, représentent l'oxygène ou un radical -NZ-;
- les radicaux Z, identiques ou différents, désignent l'hydrogène ou un radical alkyle en C₁-C₁₈, linéaire ou ramifié, un radical cycloalkyle en C₅-C₁₂ pouvant être substitué par un ou plusieurs radicaux alkyle en C₁-C₄ ;
- T₃, T₄ et T₅, identiques ou différents, sont choisis parmi : l'hydrogène; un métal alcalin; un radical ammonium éventuellement substitué par un ou plusieurs radicaux alkyle ou hydroxyalkyle ; un radical alkyle linéaire ou ramifié en C₁-C₁₈ ; un radical cycloalkyle en C₅-C₁₂ éventuellement substitué par un ou plusieurs radicaux alkyle en C₁-C₄ ; un radical polyoxyéthyléné comprenant de 1 à 6 unités d'oxyde d'éthylène et dont le groupe OH terminal est méthylé; un radical de formule (3), (4) ou (5) suivantes : 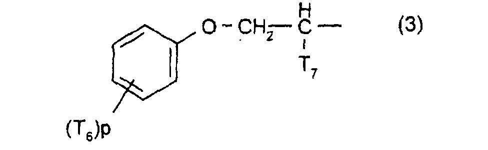 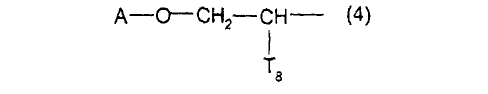 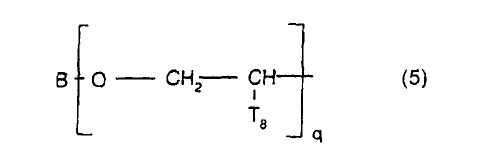
dans lesquelles :
- T₆ est l'hydrogène ou un radical méthyle ;
- T₇ est un radical alkyle en C₁-C₉ ;
- p est un nombre entier allant de 0 à 3 ;
- q est un nombre entier allant de 1 à 10 ;
- A est un radical alkyle en C₄-C₈ ou un radical cycloalkyle en C₅-C₈ ;
- B est choisi parmi : un radical alkyle linéaire ou ramifié en C₁-C₈ ; un radical cycloalkyle en C₅-C₈ ; un radical aryle éventuellement substitué par un ou plusieurs radicaux alkyle en C₁-C₄ ;
- T₈ est l'hydrogène ou un radical méthyle ;

Une première famille préférée de dérivés de 1,3,5-triazine est celle, notamment décrite dans le document EP-A-0517104 (dont les enseignements sont, pour ce qui touche à la définition même de ces produits, totalement inclus à titre de référence dans la présente description) des 1,3,5-triazines répondant à la formule (2) ci-dessus et présentant l'ensemble des caractéristiques suivantes :
- X₁, X₂ et X₃ sont identiques et représentent l'oxygène ;
- T₃ est choisi parmi : un radical cycloalkyle en C₅-C₁₂ éventuellement substitué par un ou plusieurs radicaux alkyles en C₁-C₄ ; un radical de formule (2), (3) ou (4) ci-dessus dans lesquelles :
   - B est un radical alkyle en C₁-C₄ ;
   - T₈ est le radical méthyle;
   - T₄ et T₅, identiques ou différents, sont choisis parmi : l'hydrogène ; un métal alcalin ; un radical ammonium éventuellement substitué par un ou plusieurs radicaux alkyle ou hydroxyalkyle ; un radical alkyle linéaire ou ramifié en C₁-C₁₈ ; un radical cycloalkyle en C₅-C₁₂ éventuellement substitué par un ou plusieurs radicaux alkyle en C₁-C₄ ; un radical de formule (3), (4) ou (5) ci-dessus dans lesquelles :
      - B est un radical alkyle en C₁-C₄ ;
      - T₈ est le radical méthyle.

Une deuxième famille préférée de dérivés de 1,3,5-triazine selon l'invention est celle, notamment décrite dans le document EP-A-0570838 (dont les enseignements sont, pour ce qui touche à la définition même de ces produits, totalement inclus à titre de référence dans la présente description) des 1,3,5-triazines répondant à la formule (2) et présentant l'ensemble des caractéristiques suivantes :
- X₁ est l'oxygène ; X₂ est le radical -NH- ou l'oxygène ;
- X₃ est le radical -NH- ;
- T₅ est choisi parmi : un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- T₃ est choisi parmi : l'hydrogène; un métal alcalin ; un radical ammonium; un radical de formule (5) ; un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- si X₂ est le radical -NH-, alors T₄ est choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄ ;
- si X₂ est l'oxygène, alors T₄ est choisi parmi l'hydrogène ; un métal alcalin ; un radical ammonium; un radical de formule (5) ; un radical alkyle linéaire ou ramifié en C₁-C₁₈ ; un radical cycloalkyle en C₅-C₁₂ éventuellement substitué avec un ou plusieurs radicaux alkyles en C₁-C₄.

Une troisième famille préférée de dérivés de 1,3,5-triazine selon l'invention est celle, notamment décrite dans le document EP-A-0796851 dont les enseignements sont, pour ce qui touche à la définition même de ces produits, totalement inclus à titre de référence dans la présente description) des 1,3,5-triazines répondant à la formule (2) et présentant l'ensemble des caractéristiques suivantes :
- X₁, X₂ et X₃ désignent simultanément -NZ-;
- les radicaux Z, identiques ou différents, désignent l'hydrogène ou un radical alkyle en C₁-C₁₈, linéaire ou ramifié, un radical cycloalkyle en C₅-C₁₂ pouvant être substitué par un ou plusieurs radicaux alkyle en C₁-C₄ ;
- T₃, T₄ et T₅, identiques ou différents, désignent l'hydrogène ou un radical Z.

Ces filtres UV organiques du type triazine sont décrits dans les brevets US4617390, EP517104, EP570838, EP796851 (faisant partie intégrante du contenu de la description).

Parmi ces filtres UV du type triazine de formule (2), on citera plus particulièrement:
- la 2,4,6-tris[p-(2'-éthylhexyl-1'-oxycarbonyl)-anilino]-1,3,5-triazine qui est un filtre connu en soi, actif dans l'UV-B, se présentant sous une forme solide, et qui est vendu notamment sous la dénomination commerciale de "UVINUL T150" par la Société BASF. Ce produit répond à la formule suivante : 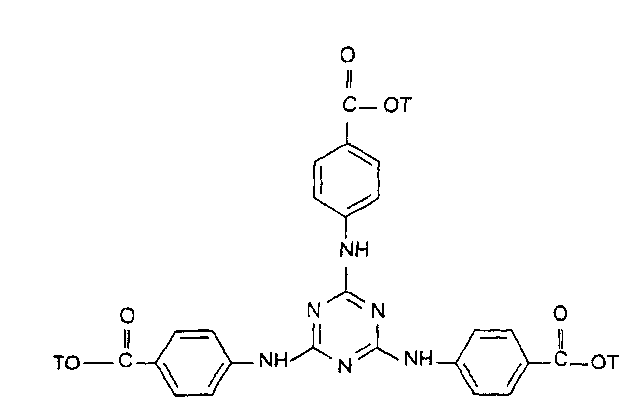
dans laquelle T désigne un radical 2-éthylhexyle ;
- la 2-[(p-(tertiobutylamido)anilino]-4,6-bis-[(p-(2'-éthylhexyl-1'-oxycarbonyl)anilino]- 1,3,5-triazine, de structure suivante : 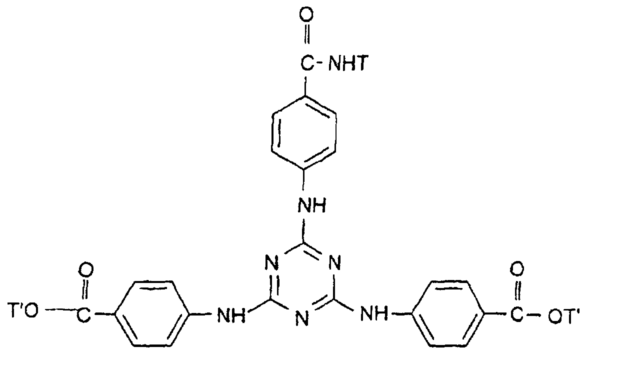
dans laquelle T' désigne un radical 2-éthylhexyle et T désigne un radical tert-butyle.

Parmi les filtres UV du type triazine conformes à l'invention, on peut également mentionner les dérivés insolubles de s-triazine portant des groupements benzalmalonates et/ou phenylcyanoacrylates tels que ceux décrits dans la demande EP-A-0790243 (faisant partie intégrante du contenu de la description).

Parmi ces filtres UV du type triazine, on citera plus particulièrement les composés suivants :
- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,
- la 2,4,6-tris(α-cyano-4-aminocinnamate d'éthyle)-s-triazine.

Parmi les filtres UV du type triazine conformes à l'invention, on peut également mentionner ceux répondant à la formule (6) suivante : 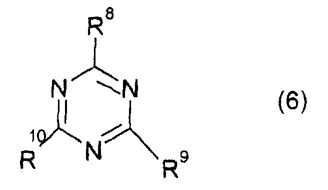 dans laquelle R⁸, R⁹, R¹⁰, indépendamment, sont phenyle, phenoxy, pyrrolo, dans lesquels les phenyle, phenoxy, pyrrolo sont éventuellement substitués par un, deux ou trois substituants choisis parmi OH, C₁-C₁₈alkyle ou alkoxy, C₁-C₁₈carboxyalkyle, C₅-C₈cycloalkyle, un groupe méthylidènecamphre, un groupe -(CH=CH)ₙ(CO)-OR⁴, avec R⁴ soit C₁-C₁₈alkyle soit cinnamyle, et n vaut 0 ou 1.

Ces composés sont décrits dans WO 97/03642, GB 2286774, EP-743309, WO 98/22447, GB 2319523 (faisant partie intégrante du contenu de la description).

Parmi les filtres UV du type triazine conformes à l'invention, on peut encore mentionner les dérivés insolubles de s-triazine portant des groupements benzotriazoles et/ou benzothiazoles tels que ceux décrits dans la demande WO98/25922 (faisant partie intégrante du contenu de la description).

Parmi ces composés, on peut citer plus particulièrement :
- la 2,4,6-tris[(3'-benzotriazol-2-yl-2'-hydroxy-5'-methyl) phenylamino]-s-triazine,
- 2,4,6-tris[(3'-benzotriazol-2-yl-2'-hydroxy-5'-ter-octyl) phénylamino]-s-triazine.

Parmi les filtres UV organiques du type triazole conformes à l'invention, on peut citer ceux de formule suivante (7) tels que décrits dans la demande WO95/22959 (faisant partie intégrante du contenu de la description) : 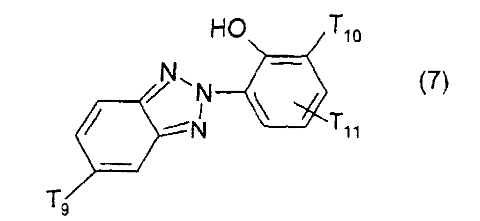 dans laquelle T₉ désigne un atome d'hydrogène ou un radical alkyle en C₁-C₁₈; T₁₀ et T₁₁, identiques ou différents, désignent un radical alkyle en C₁-C₁₈ éventuellement substitué par un phényle.

A titre d'exemple de composés de formule (7), on peut citer les produits commerciaux TINUVIN 328, 320, 234 et 350 de la Société CIBA-GEIGY de structure suivante :

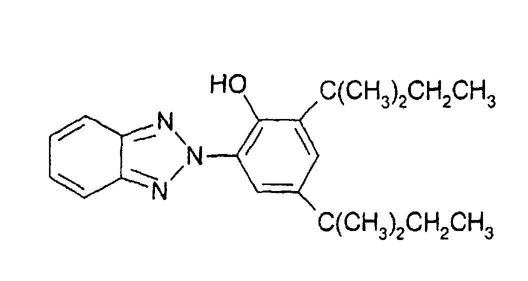

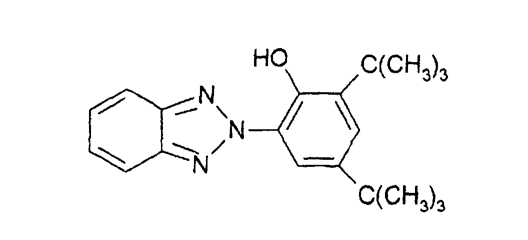

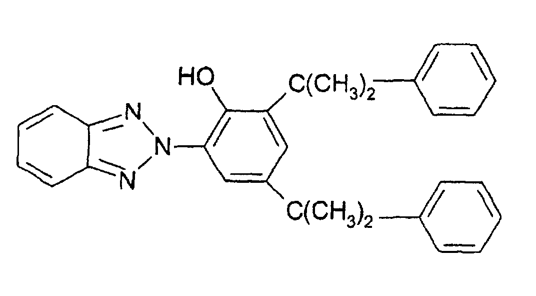

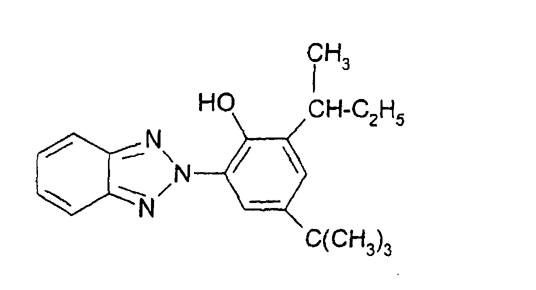

Parmi les filtres UV organiques du type triazole conformes à l'invention, on peut citer les composés tels que décrits dans les brevets US 5 687 521, US 5 687 521, US 5 373 037, US 5 362 881 et en particulier le [2 ,4'-dihydroxy-3-(2H-benzotriazol-2-yl)-5-(1,1,3,3-tétraméthylbutyl)-2'-n-octoxy-5'-benzoyl] diphénylméthane vendu sous le nom MIXXIM PB30 par la société FAIRMOUNT CHEMICAL de structure :

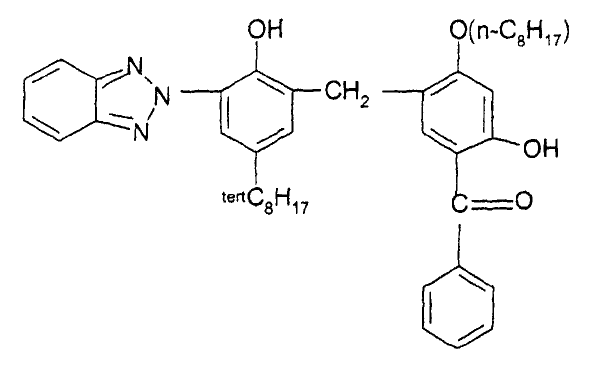

Parmi les filtres UV organiques du type benzotriazole conformes à l'invention, on peut citer les dérivés de méthylène bis-(hydroxyphényl benzotriazole) de structure suivante : 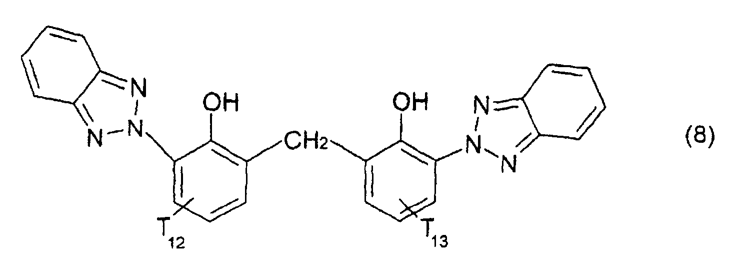 dans laquelle les radicaux T₁₂ et T₁₃, identiques ou différents, désignent un radical alkyle en C₁-C₁₈ pouvant être substitué par un ou plusieurs radicaux choisis parmi alkyle en C₁-C₄, cycloalkyle en C₅-C₁₂ ou un reste aryle. Ces composés sont connus en soi et décrits dans les demandes US 5237 071, US 5 166 355, GB-A-2 303 549, DE 197 26 184 et EP-A-893 119 (faisant partie intégrante de la description).

Dans la formule (8) définie ci-dessus : les groupes alkyle en C₁-C₁₈ peuvent être linéaires ou ramifiés et sont par exemple méthyle, éthyle, n-propyle, isopropyle, n-butyle, isobutyle, tert-butyle, tert-octyle, n-amyle, n-hexyle, n-heptyle, n-octyle, iso-octyle, n-nonyle, n-décyle, n-undécyle, n-dodécyle, tétradécyle, hexydécyle, ou octadécyle; les groupes cycloalkyle en C₅-C₁₂ sont par exemple cyclopentyle, cyclohexyle, cyclooctyle; les groupes aryle sont par exemple phényle, benzyle.

Parmi les composés de formule (8), on préfère plus particulièrement ceux de structure suivante :

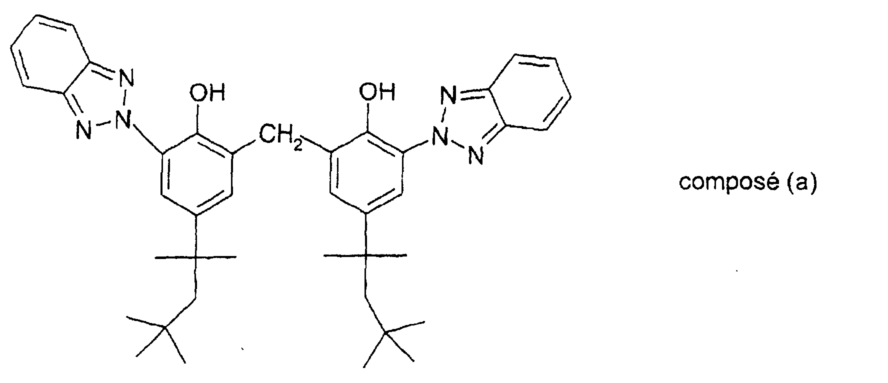

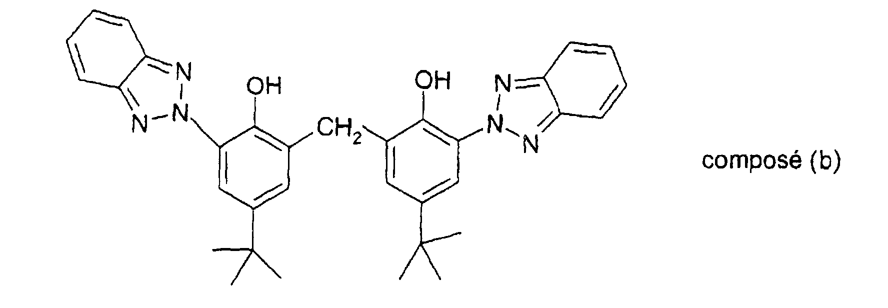

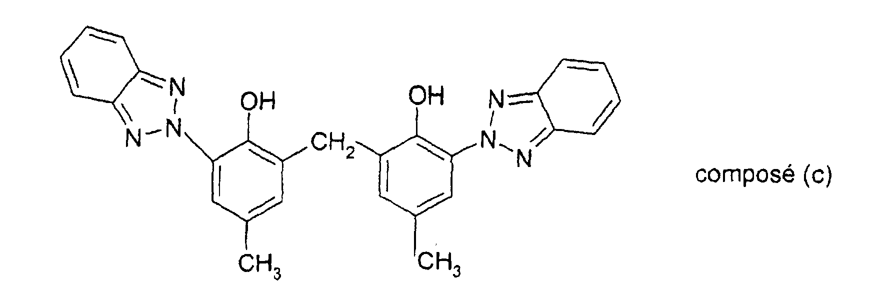

Le composé (a) de nomenclature 2,2'-méthylène-bls-[6-(2H-benzotriazol-2-yl)-4-(1,1,3,3-tetraméthylbutyl)phénol] est vendu sous forme pure sous le nom MIXXIM BB/100 par le société FAIRMOUNT CHEMICAL et sous forme micronisée sous le nom TINOSORB M par CIBA GEIGY.

Le composé (c) de nomenclature 2,2'-méthylène-bis-[6-(2H-benzotriazol-2-yl)-4-(methyl)phénol] est vendu sous le nom MIXXIM BB/200 par le société FAIRMOUNT CHEMICAL.

Parmi les filtres organiques insolubles du type amide vinylique, on peut citer par exemple les composés de formules suivante qui sont décrits dans la demande WO95/22959 (faisant partie intégrante du contenu de la description):

T₁₄-(Y)r-C(=O)-C(T₁₅)=C(T₁₆)-N(T₁₇)(T₁₈) (9)

dans laquelle T₁₄ est un radical alkyle en C₁-C₁₈, de préférence en C₁-C₅ ou un groupe phényle éventuellement substitué par un, deux ou trois radicaux choisis parmi OH, alkyle en C₁-C₁₈, alcoxy en C₁-C₈, ou un groupe -C(=O)-OT₁₉ où T₁₉ est un alkyle en C₁-C₁₈ ; T₁₅, T₁₆, T₁₇ et T₁₈ identiques ou différents désignent un radical alkyle en C₁-C₁₈, de préférence en C₁-C₅ ou un atome d'hydrogène ; Y est N ou O et r vaut 0 ou 1.

Parmi ces composés, on citera plus particulièrement :
- la 4-octylamino-3-pentèn-2-one ;
- l'éthyl-3-octylamino-2-buténoate ;
- la 3-octylamino-1-phényl-2-butèn-1-one
- la 3-dodecylamino-1-phenyl-2-buten-1-one.

Parmi les filtres organiques du type cinnamamide conformes à l'invention, on peut citer également les composés tels que décrits dans la demande WO95/22959 (faisant partie intégrante du contenu de la description) et répondant à la structure suivante : 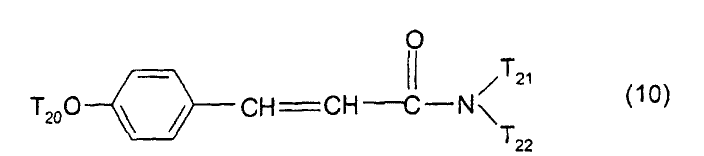 dans laquelle OT₂₀ est un radical hydroxy ou alcoxy en C₁-C₄, de préférence méthoxy ou éthoxy; T₂₁ est hydrogène, alkyle en C₁-C₄, de préférence méthyle ou éthyle ; T₂₂ est un groupe -(CONH)s-phényle ou s vaut 0 ou 1 et le groupe phényle peut être substitué par un, deux ou trois groupes choisis parmi OH, alkyle en C₁-C₁₈, alcoxy en C₁-C₈, ou un groupe -C(=O)-OT₂₃ où T₂₃ est un alkyle en C₁-C₁₈ et plus préférentiellement T₂₃ est un groupe phényle, 4-méthoxyphényle ou phénylaminocarbonyle.

On peut également citer les dimères cinnamamides tels que ceux décrits dans le brevet US 5888481 comme par exemple le composé de structure :

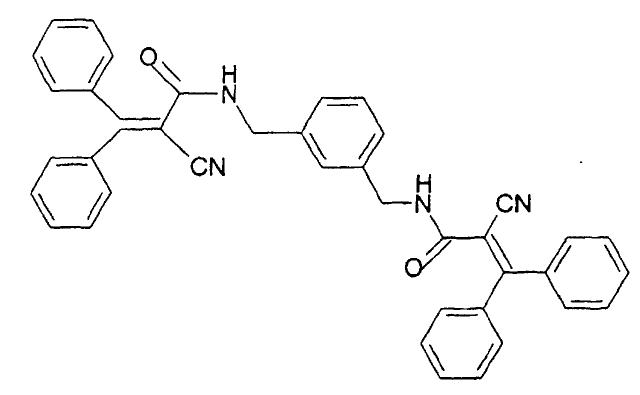

Une autre famille particulière de filtres organiques insolubles conformes à l'invention sont les sels de métaux polyvalents (par exemple Ca²⁺, Zn²⁺, Mg²⁺, Ba²⁺, Al³⁺ ou Zr⁴⁺) de filtres organiques sulfoniques ou carboxyliques tels que les sels de métaux polyvalents de dérivés sulfonés de benzylidène camphre tels que ceux décrits dans la demande FR-A 2 639 347; les sels de métaux polyvalents de dérivés sulfonés de benzimidazole tels que ceux décrits dans la demande EP-A-893 119; les sels de métaux polyvalents de dérivés d'acide cinnamique tels que ceux décrits dans la demande JP-87 166 517.

On peut également citer les complexes de métaux ou d'ammonium ou d'ammonium substitué de filtres organiques UV-A et/ou UV-B tels que décrits dans les demandes de brevet WO93/10753, WO93/11095 et WO95/05150.

Le ou les filtres organiques insolubles sous forme micronisée selon l'invention sont généralement présents dans les compositions filtrantes selon l'invention à une concentration totale comprise entre 0,1 et 15 % en poids environ, et de préférence entre 0,2 et 10 % en poids environ, par rapport au poids total de la composition.

Les filtres organiques insolubles sous forme micronisée selon l'invention peuvent être amenés sous la forme particulaire souhaitée par tout moyen ad-hoc tel que notamment broyage à sec ou en milieu solvant, tamisage, atomisation, micronisation, pulvérisation. La taille moyenne des particules varie de 0,01 à 2µm et plus préférentiellement de 0,02 à 1,5 µm et plus particulièrement de 0,03 à 1,0 µm.

Les filtres organiques insolubles selon l'invention sous forme micronisée. peuvent être obtenus par un procédé de broyage d'un filtre UV insoluble sous forme de particules de taille grossière en présence d'un tensio-actif approprié permettant d'améliorer la dispersion des particules ainsi obtenues dans les formulations cosmétiques.

Un exemple de procédé de micronisation de filtres organiques insolubles est décrit dans les demandes GB-A-2 303 549 et EP-A-893 119 faisant partie intégrante de la description. L'appareil de broyage utilisé selon ces documents peut être un broyeur à jet, à billes, à vibration ou à marteau et de préférence un broyeur à haute vitesse d'agitation ou un broyeur à impact et plus particulièrement un broyeur à billes rotatives, un broyeur vibrant, à broyeur à tube ou un broyeur à tige.

Selon ce procédé particulier, on utilise à titre de tensio-actifs pour le broyage desdits filtres, les alkylpolyglucosides de structure CₙH ₂ₙ₊₁ O(C₆H₁₀O₅)ₓH dans laquelle n est un entier de 8 à 16 et x est le degré moyen de polymérisation de l'unité (C₆H₁₀O₅) et varie de 1,4 à 1,6. Ils peuvent être choisis parmi des esters en C₁-C₁₂ d'un composé de structure CₙH₂ₙ₊₁ O(C₆H₁₀O₅)ₓH et plus précisément un ester obtenu par réaction d'un acide carboxylique en C₁-C₁₂ tel que l'acide formique, acétique, propionique, butyrique, sulfosuccinique, citrique ou tartrique avec une ou plusieurs fonctions OH libres sur l'unité glucoside (C₆H₁₀O₅). Lesdits tensio-actifs sont utilisés en général à une concentration de allant de 1 à 50% en poids et plus préférentiellement de 5 à 40% en poids par rapport au filtre insoluble dans sa forme micronisée.

Un autre objet de l'invention consiste en des compositions cosmétiques ou dermatologiques caractérisées par le fait qu'elle comprennent au moins une émulsion telle que définie précédemment.

Les compositions cosmétiques antisolaires selon l'invention peuvent bien entendu contenir un ou plusieurs filtres organiques complémentaires actifs dans l'UVA et/ou l'UVB (absorbeurs), solubles dans au moins l'une des phases des compositions. Ces filtres complémentaires peuvent être notamment choisis parmi les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP 933376; les dérivés de la benzophénone ; les dérivés de β,β'-diphénylacrylate, ; les dimères dérivés d'α-alkylstyrène tels que ceux décrits dans la demande DE 198 55 649; les dérivés de benzimidazole; les dérivés bis-benzoazolyle tels que décrits dans les brevets EP-A-0669323 et US 2,463,264; les dérivés de l'acide p-aminobenzoïque; les polymères filtres et silicones filtres tels que ceux décrits notamment dans la demande WO-93/04665.

Comme exemples de filtres solaires complémentaires actifs dans l'UV-A et/ou l'UV-B, on peut citer :
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-ter-butyl-4'-méthoxy-dibenzoylméthane,
le 4-isopropyl-dibenzoylméthane,
le 4-méthoxy cinnamate de 2-éthylhexyle,
le diisopropyl cinnamate de méthyle,
le 4-méthoxy cinnamate d'isoamyle,
le 4-méthoxy cinnamate de diéthanolamine,
l'anthranilate de menthyle,
le 2-éthylhexyl-2-cyano-3,3'-diphénylacrylate,
l'éthyl-2-cyano-3,3'-diphénylacrylate,
l'acide 2-phényl benzimidazole 5-sulfonique et ses sels,
le 3-(4'-triméthylammonium)-benzylidèn-bornan-2-on-méthylsulfate,
le 2-hydroxy-4-méthoxybenzophénone,
le 2-hydroxy-4-méthoxybenzophénone-5-sulfonate,
le 2,4-dihydroxybenzophénone,
le 2,2',4,4'-tétrahydroxybenzophénone,
le 2,2-dihydroxy-4,4'diméthoxybenzophénone,
le 2-hydroxy-4-n-octoxybenzophénone,
le 2-hydroxy-4-méthoxy-4'-méthylbenzophénone,
l'acide α-(2-oxoborn-3-ylidène)-tolyl-4-sulfonique et ses sels solubles
le 3-(4'-sulfo)benzyiidèn-bornan-2-one et ses sels solubles,
le 3-(4'méthylbenzylidène)-d,l-camphre,
le 3-benzylidène-d,l-camphre,
l'acide benzène 1,4-di(3-méthylidène-10-camphosulfonique) et ses sels solubles,
l'acide urocanique,
la 2,4-bis {[4-2-éthyl-hexyloxy}]-2-hydroxy]-phenyl}-6-(4-méthoxy-phenyl)-1,3,5-triazine ;
le polymère de N-(2 et 4)-[(2-oxobom-3-ylidèn)méthyl]benzyl]-acrylamide,
l'acide 1,4-bis-benzimidazolyl-phénylen-3,3',5,5'-tétrasulfonique et ses sels solubles
les polyorganosiloxanes à fonction benzalmalonate
les polyorganosiloxanes à fonction benzotriazole tel que le Dimétrizole Trisiloxane.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions de l'invention peuvent comprendre en outre des adjuvants cosmétiques classiques notamment choisis parmi les corps gras, les solvants organiques, les épaississants, les adoucissants, les opacifiants, les stabilisants, les émollients, les agents anti-mousse, les agents hydratants, les parfums, les conservateurs, les polymères, les charges, les séquestrants, les propulseurs, les agents alcalinisants ou acidifiants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les polyoléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Bien entendu, l'homme de l'art veillera à choisir ce ou ces éventuels composés complémentaires et/ou leurs quantités de manière telle que les propriétés avantageuses, en particulier la rémanence à l'eau, la stabilité, attachées intrinsèquement aux émulsions conformes à l'invention ne soient pas, ou substantiellement pas, altérées par la ou les adjonctions envisagées.

Les compositions de l'invention peuvent être préparées selon les techniques bien connues de l'homme de l'art, destinées à la préparation eau-dans-huile.

Ces compositions peuvent se présenter en particulier sous forme d'émulsion, simple ou complexe (H/E/H ou E/H/E) telle qu'une crème, un lait, un gel ou un gel crème, de poudre, de bâtonnet solide et éventuellement être conditionnée en aérosol et se présenter sous forme de mousse ou de spray.

La phase aqueuse peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme de crème ou d'un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

Comme indiqué en début de description, un autre objet de la présente invention réside dans l'utilisation d'une émulsion selon l'invention pour la fabrication de compositions cosmétiques pour la protection de la peau et/ou des cheveux contre le rayonnement ultraviolet, en particulier le rayonnement solaire.

Un autre objet de la présente invention réside dans l'utilisation silicone organomodifiée non-filtrante dans la fabrication d'une émulsion eau-dans-huile cosmétique ou dermatologique photoprotectrice, contenant au moins un filtre UV organique insoluble dans l'émulsion et sous forme micronisée, en vue d'améliorer la résistance à l'eau de son pouvoir filtrant (rémanence à l'eau).

Des exemples concrets, mais nullement limitatifs, illustrant l'invention, vont maintenant être donnés.

### EXEMPLE

| **COMPOSITION Emulsion E/H** | **Poids en grammes** |
|---|---|
| Poly diméthyl/méthylcétyl méthylsiloxane oxyéthyléné (ABIL EM 90D - GOLDSCHMIDT) | 2 |
| Phényl triméthylsiloxy trisiloxane (DOW CORNING 556 COSMETIC grade fluid - DOW CORNING) | 3 |
| Benzoate d'alcools en C12/C15 (WITCONOL TN - WITCO) | 8 |
| Methylène bis-(tetraméthylbutyl hydroxyphényl benzotriazole) sous forme micronisée vendue sous le nom TINOSORB M Taille moyenne des particules 150 nm à 200 nm. | 5 |
| Drometrizole Trisiloxane | 2 |
| 2,4-bis{[4-2-éthyl-hexyloxy)]-2-hydroxy]-phényl}-6-(4-méthoxyphényl)-1,3,5-triazine | 2 |
| Oxyde de titane (TITANIUM DIOXYDE MT100 TV - TAYCA) | 3 |
| Glycérine | 5 |
| Sulfate de magnésium | 0.7 |
| Conservateurs | qs |
| Eau déminéralisée qsp | 100 g |

## Revendications

1. Emulsion eau-dans-huile cosmétique ou dermatologique, **caractérisée par le fait qu'**elle comporte :
(a) au moins une phase aqueuse et
(b) au moins une phase grasse ;
(c) au moins un système photoprotecteur capable de filtrer les rayons UV contenant au moins un filtre UV organique insoluble dans l'émulsion, sous forme micronisée dont la taille moyenne des particules varie de 0,01 à 2µm ;
(d) au moins une silicone organomodifiée non-filtrante comportant des groupements oxyalkylénés ;
sous réserve que ledit filtre UV organique insoluble soit différent de la 2,4,6-tris[p-(2'-éthylhexyl-1'-oxycarbonyl)-anilino]-1,3,5-triazine sous forme insoluble micronisée et du composé de structure : 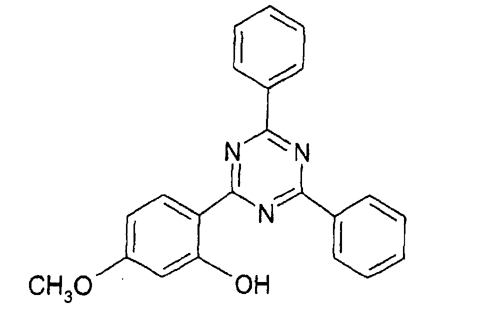 sous forme insoluble micronisée.

2. Emulsion selon la revendication 1, où la silicone organomodifiée non-filtrante se présente sous forme d'huile, de cire, de résine ou de gomme, hydrosoluble ou insoluble dans l'eau.

3. Emulsion selon la revendication 1, où la silicone organomodifiée non-filtrante est choisie parmi les polyorganosiloxanes comportant des groupements oxyéthylénés et/ou oxypropylénés

4. Emulsion selon l'une quelconque des revendications 1 à 3, où la silicone organomodifiée non-filtrante répond à la formule suivante : 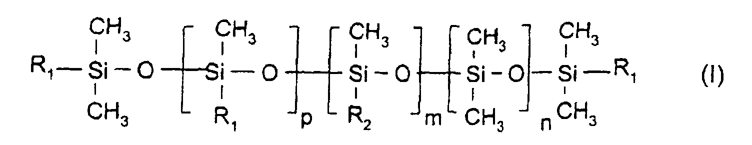
- les radicaux R₁, identiques ou différents, représente un radical alkyle, linéaire ou ramifié, en C₁-C₃₀ ou phényle.
- les radicaux R₂, identiques ou différents, désignent un groupe -C_{c}H_{2c}-(-O-C₂H₄)ₐ-(-O-C₃H₆)_{b}-(O-C₄H₈)_{d}-R₃,
- les radicaux R₃, identiques ou différents, sont choisis parmi un atome d'hydrogène, un radical hydroxyle, un radical alkyle linéaire ou ramifié de 1 à 12 atomes de carbone, un radical alcoxy linéaire ou ramifié de 1 à 6 atomes de carbone, un radical acyloxy linéaire ou ramifié de 2 à 12 atomes de carbone, NHCH₂CH₂COOM, aminoalkyle éventuellement substitué sur l'amine, carboxyacyle en C₁-C₃₀, un groupement phosphono éventuellement substitué,
-O-CO-(CH₂)_{d}-CO₂M, -NHCO(CH₂)_{d}OH, -NH₃Y.
- M, identique ou différent, désigne un atome d'hydrogène, Na, K, Li, NH₄ ou une amine organique, Y représente un anion minéral ou organique monovalent tel que halogénure (chlorure, bromure), sulfate, carboxylate (acétate, lactate, citrate).
- a varie de 0 à 100,
- b varie de 0 à 50,
- c varie de 0 à 5,
- a + b est supérieur ou égal à 1,
- d varie de 0 à 10,
- m varie de 0 à 20,
- n varie de 0 à 500,
- p varie de 0 à 50,

5. Emulsion selon la revendication 4 , où la silicone organomodifiée non-filtrante de formule (I) est choisie parmi celles pour lesquelles les radicaux R₁ représentent tous des radicaux méthyle et le radical R₃ représente un radical hydroxy.

6. Emulsion selon la revendication 5 , où la silicone organomodifiée non-filtrante de formule (I) est le poly diméthyl / méthyl siloxane oxyéthyléné oxypropyléné (OE/OP 18/18) pour lequel p+n est 396 et m est 4, (nom CTFA : Cyclométhicone 90% Dimethicone copolyol 10%)

7. Emulsion selon la revendication 4 , où la silicone organomodifiée non-filtrante de formule (I) est choisie parmi celles pour lesquelles les radicaux R₁ représentent des radicaux méthyle et des radicaux alkyls en C₂-C₃₀ et le radical R₃ représente un radical hydroxy.

8. Emulsion selon la revendication 7 , où la silicone organomodifiée non-filtrante de formule (I) est le poly méthyllauryl / méthyl siloxane oxyéthyléné oxypropyléné (OE/OP 18/18) pour lequel p+n est 35 et m est 3, (nom CTFA : Laurylmethicone copolyol 91%, Isostearyl alcohol 9%).

9. Emulsion selon la revendication 7, où la silicone organomodifiée non-filtrante de formule (I) est le poly méthylcetyl / méthyl siloxane oxyéthyléné oxypropyléné, pour lequel p est 20 à 25 et m est 5 et n est 75 (nom CTFA : cetyldimethicone copolyol).

10. Emulsion selon l'une quelconque des revendications 1 à 3, où la silicone organomodifiée non-filtrante est une silicone oxyalkylénée substituée en α-ω à structure linéaire, substituée aux deux extrémités de la chaîne principale par des groupements oxyalkylénés reliés aux atomes de Si par l'intermédiaire d'un groupement hydrocarboné.

11. Emulsion selon la revendication 10 , où la silicone organomodifiée non-filtrante est une silicone répondant à la formule générale (II) suivante : 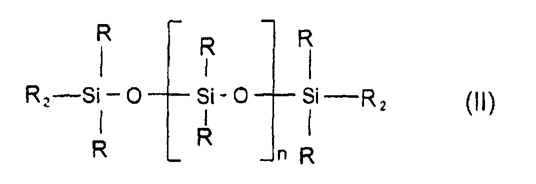 dans laquelle n varie de 1 à 500 ; les radicaux R₂, identiques ou différents, désignent un groupe -C_{c}H_{2c}-(-O-C₂H₄)ₐ-(-O-C₃H₆)_{b}-(O-C₄H₈)_{d}-R₃ où a, b, c et R₃ ont les mêmes définitions indiquées dans la formule (I) et les radicaux R représentent, identiques ou différents un radical alkyle, linéaire ou ramifié, en C₁-C₃₀ ou phényle.

12. Emulsion selon la revendication 11, où tous les radicaux R sont des radicaux méthyles ; c va de 2 à 4, a va de 3 à 100, b va de 1 à 50, d vaut 0, R₃ est un radical hydroxy , n va de 50 à 200.

13. Emulsion selon la revendication 11 ou 12, où le rapport en poids des unités C₂H₄O par rapport aux unités C₃H₆O va de 100:10 à 20:80.

14. Emulsion selon l'une quelconque des revendications 1 à 13, dans laquelle la ou les silicones organomodifiées non-filtrantes sont présentes dans des quantités allant de 0,1 à 20 % par rapport au poids total de l'émulsion, de préférence en une quantité allant de 0,5 à 10 %.

15. Emulsion selon l'une quelconque des revendications 1 à 14, où le ou les filtres UV organiques insolubles micronisés sont choisis parmi les filtres UV organiques du type oxanilide, du type triazine, du type triazole, du type amide vinylique ou du type cinnamamide.

16. Emulsion selon la revendication 15, où les filtres UV du type oxanilide répondent à la structure : 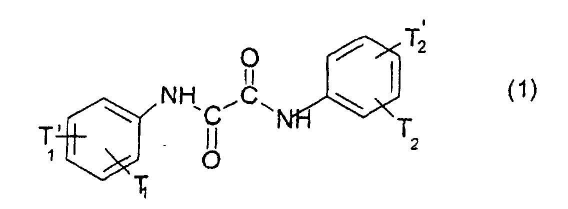 dans laquelle T₁, T'₁, T₂ et T'₂ désignent, identiques et différents, un radical alkyle en C₁-C₈ ou un radical alcoxy en C₁-C₈.

17. Emulsion selon la revendication 15, où les filtres UV du type triazine sont choisis parmi ceux répondant à la formule générale suivante : 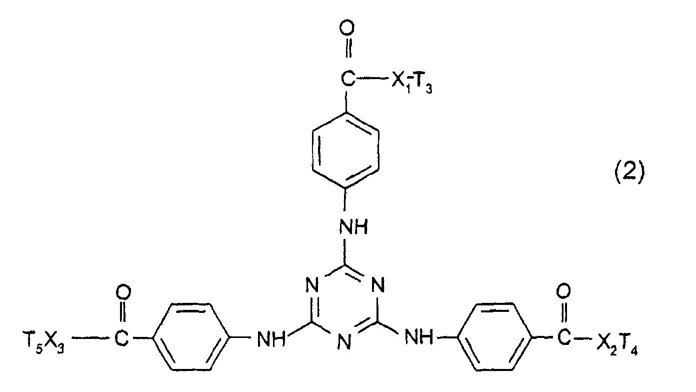 dans laquelle :
- X₁, X₂ et X₃, identiques ou différents, représentent l'oxygène ou un radical -NZ-;
- les radicaux Z, identiques ou différents, désignent l'hydrogène ou un radical alkyle en C₁-C₁₈, linéaire ou ramifié, un radical cycloalkyle en C₅-C₁₂ pouvant être substitué par un ou plusieurs radicaux alkyle en C₁-C₄ ;
- T₃, T₄ et T₅, identiques ou différents, sont choisis parmi : l'hydrogène; un métal alcalin; un radical ammonium éventuellement substitué par un ou plusieurs radicaux alkyle ou hydroxyalkyle ; un radical alkyle linéaire ou ramifié en C₁-C₁₈; un radical cycloalkyle en C₅-C₁₂ éventuellement substitué par un ou plusieurs radicaux alkyle en C₁-C₄ ; un radical polyoxyéthyléné comprenant de 1 à 6 unités d'oxyde d'éthylène et dont le groupe OH terminal est méthylé ; un radical de formule (3), (4) ou (5) suivantes : 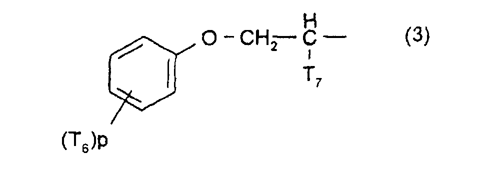 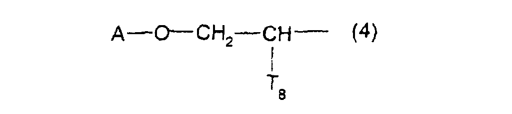 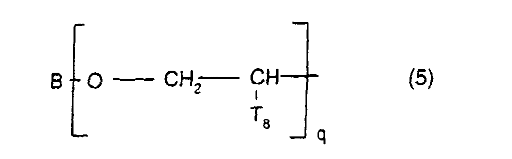
dans lesquelles :
- T₆ est l'hydrogène ou un radical méthyle ;
- T₇ est un radical alkyle en C₁-C₉ ;
- p est un nombre entier allant de 0 à 3 ;
- q est un nombre entier allant de 1 à 10 ;
- A est un radical alkyle en C₄-C₈ ou un radical cycloalkyle en C₅-C₈ ;
- B est choisi parmi : un radical alkyle linéaire ou ramifié en C₁-C₈ ; un radical cycloalkyle en C₅-C₈ ; un radical aryle éventuellement substitué par un ou plusieurs radicaux alkyle en C₁-C₄ ;
- T₈ est l'hydrogène ou un radical méthyle.

18. Emulsion selon la revendication 17, où le filtre UV du type triazine répond à la formule suivante : 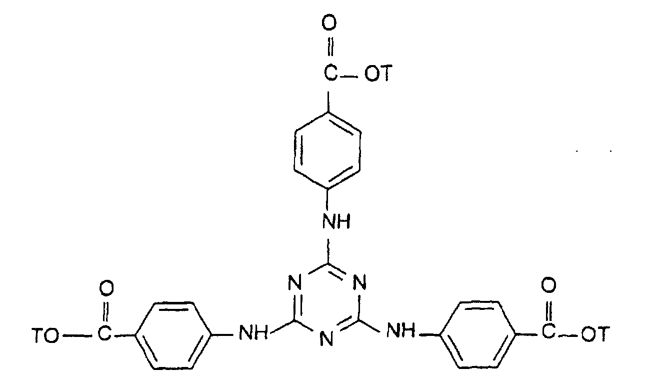 dans laquelle T désigne un radical 2-éthyfhexyle ;

19. Emulsion selon la revendication 17, où le filtre UV du type triazine répond à la formule suivante : 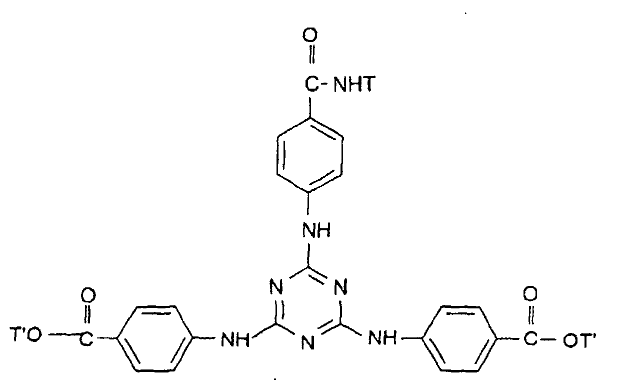 dans laquelle T' désigne un radical 2-éthylhexyle et T désigne un radical tert-butyle.

20. Emulsion selon la revendication 15, où les filtres UV du type triazine sont choisis parmi les dérivés insolubles de s-triazine portant des groupements benzalmalonates et/ou phenylcyanoacrylates.

21. Emulsion selon la revendication 20, où les filtres UV du type triazine sont choisis parmi les composés suivants :
- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,
- la 2,4,6-tris(α-cyano-4-aminocinnamate d'éthyle)-s-triazine.

22. Emulsion selon la revendication 15, où les filtres UV du type triazine sont choisis parmi ceux répondant à la formule suivante : 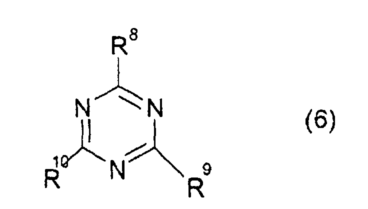 dans laquelle R⁸, R⁹, R¹⁰, indépendamment, sont phenyle, phenoxy, pyrrolo, dans lesquels les phenyle, phenoxy, pyrrolo sont éventuellement substitués par un, deux ou trois substituants choisis parmi OH, C₁-C₁₈alkyle ou alkoxy, C₁-C₁₈carboxyalkyle, C₅-C₈cycloalkyle, un groupe méthylidènecamphre, un groupe -(CH=CH)ₙ(CO)-OR⁴, avec R⁴ soit C₁-C₁₈alkyle soit cinnamyle, et n vaut 0 ou 1.

23. Emulsion selon la revendication 15, où les filtres UV du type triazine sont choisis parmi les dérivés insolubles de s-triazine portant des groupements benzotriazoles et/ou benzothiazoles.

24. Emulsion selon la revendication 23, où les filtres UV insolubles du type triazine sont choisis parmi
- la 2,4,6-tris[(3'-benzotriazol-2-yl-2'-hydroxy-5'-methyl)phenylamino]-s-triazine,
- 2,4,6-tris[(3'-benzotriazol-2-yl-2'-hydroxy-5'-ter-octyl) phénylamino]-s-triazine.

25. Emulsion selon la revendication 15, où les filtres UV organiques du type triazole répondent à la formule (7) suivante : 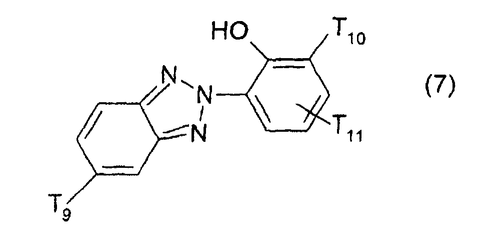 dans laquelle T₉ désigne un atome d'hydrogène ou un radical alkyle en C₁-C₁₈; T₁₀ et T₁₁, identiques ou différents, désignent un radical alkyle en C₁-C₁₈ éventuellement substitué par un phényle.

26. Emulsion selon la revendication 25, où le composé de formule (7) est choisi parmi les composés suivants :

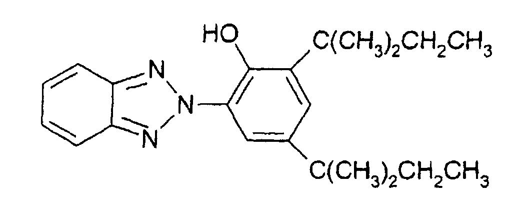

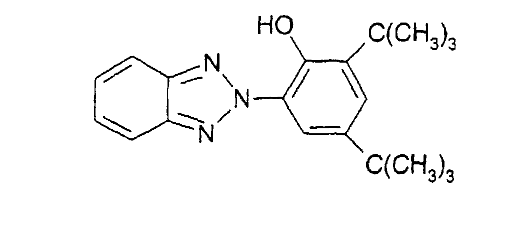

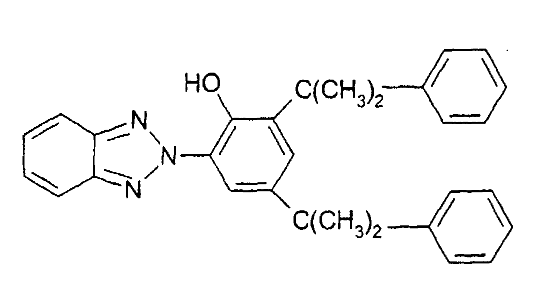

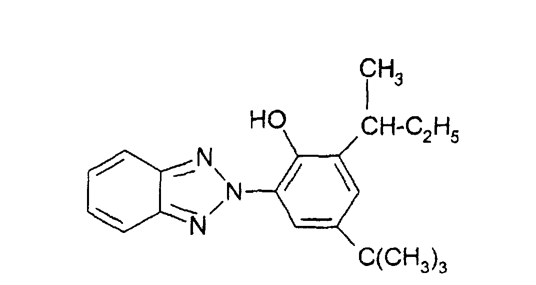

27. Emulsion selon la revendication 15, où le filtre UV insoluble sous forme micronisé est le [2 ,4'-dihydroxy-3-(2H-benzotriazol-2-yl)-5-(1,1,3,3-tétraméthylbutyl)-2'-n-octoxy-5'-benzoyl] diphénylméthane de structure :

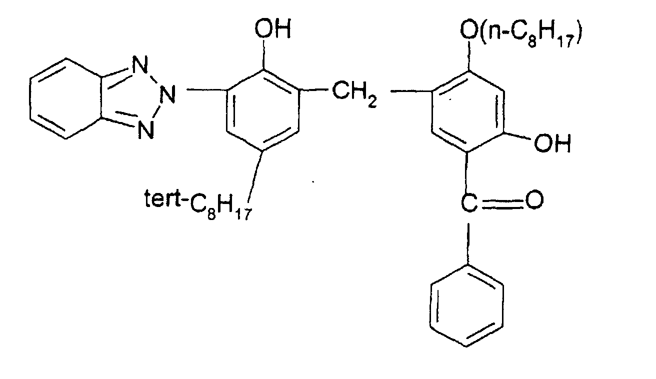

28. Emulsion selon la revendication 15, où les filtres UV organiques du type triazole sont choisis parmi les dérivés de méthylène bis-(hydroxyphényl benzotriazole) de structure suivante : 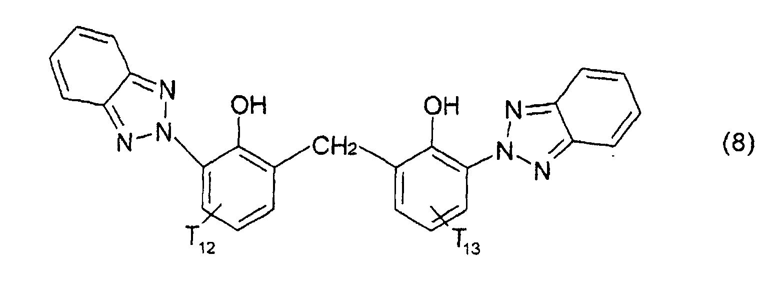 dans laquelle les radicaux T₁₂ et T₁₃, identiques ou différents, désignent un radical alkyle en C₁-C₁₈ pouvant être substitué par un ou plusieurs radicaux choisis parmi alkyle en C₁-C₄, cycloalkyle en C₅-C₁₂ ou un reste aryle.

29. Emulsion selon la revendication 28, où le composé de formule (8) est choisi dans le groupe constitué par les composés de structure suivante :

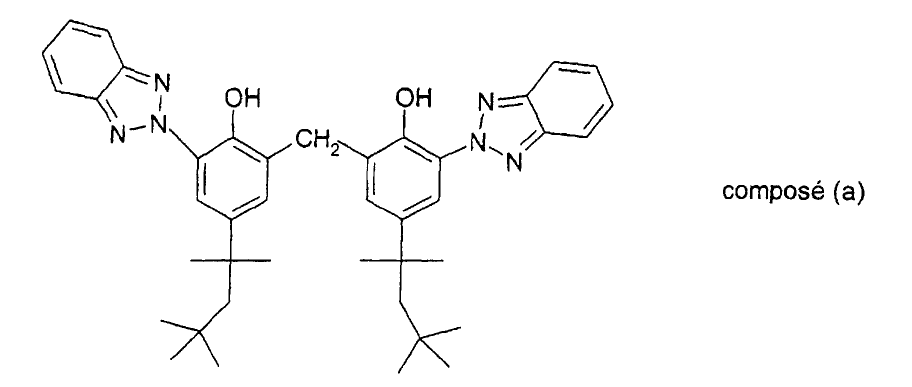

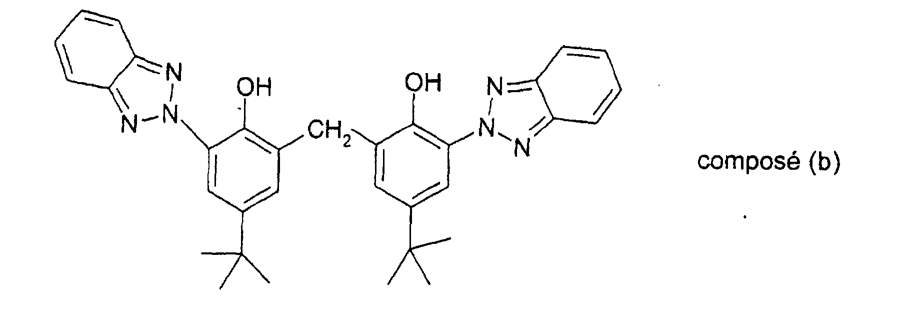

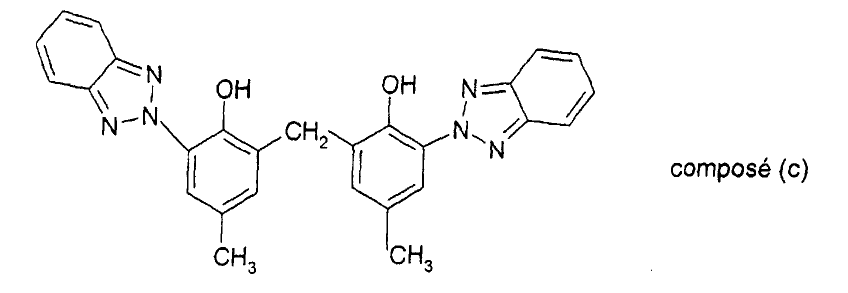

30. Emulsion selon la revendication 15, où les filtres organiques du type amide vinylique, répondent à la formule suivante :
T₁₄-(Y)r-C(=O)-C(T₁₅)=C(T₁₆)-N(T₁₇)(T₁₈) (9)
dans laquelle T₁₄ est un radical alkyle en C₁-C₁₈, de préférence en C₁-C₅ ou un groupe phényle éventuellement substitué par un, deux ou trois radicaux choisis parmi OH, alkyle en C₁-C₁₈, alcoxy en C₁-C₈, ou un groupe -C(=O)-OT₁₉ où T₁₉ est un alkyle en C₁-C₁₈ ; T₁₅, T₁₆, T₁₇ et T₁₈ identiques ou différents désignent un radical alkyle en C₁-C₁₈, de préférence en C₁-C₅ ou un atome d'hydrogène ; Y est N ou O et r vaut 0 ou 1.

31. Emulsion selon la revendication 30, où les composés de formule (9) sont choisis parmi:
- la 4-octylamino-3-pentèn-2-one ;
- l'éthyl-3-octylamino-2-buténoate ;
- la 3-octylamino-1-phényl-2-butèn-1-one
- la 3-dodecylamino-1-phenyl-2-buten-1-one.

32. Emulsion selon la revendication 15, où les filtres organiques du type cinnamamide répondent à la formule suivante : 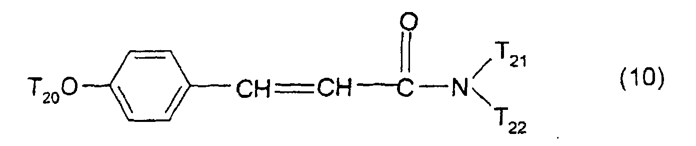 dans laquelle OT₂₀ est un radical hydroxy ou alcoxy en C₁-C₄, de préférence méthoxy ou éthoxy ; T₂₁ est hydrogène, alkyle en C₁-C₄, de préférence méthyle ou éthyle ; T₂₂ est un groupe -(CONH)s-phényle ou s vaut 0 ou 1 et le groupe phényle peut être substitué par un, deux ou trois groupes choisis parmi OH, alkyle en C₁-C₁₈, alcoxy en C₁-C₈, ou un groupe -C(=O)-OT₂₃ où T₂₃ est un alkyle en C₁-C₁₈ et plus préférentiellement T₂₃ est un groupe phényle, 4-méthoxyphényle ou phénylaminocarbonyle.

33. Emulsion selon la revendication 15, où le filtre UV insoluble est un dimère cinnamamide.

34. Emulsion selon la revendication 33, où le filtre UV insoluble est le composé de structure :

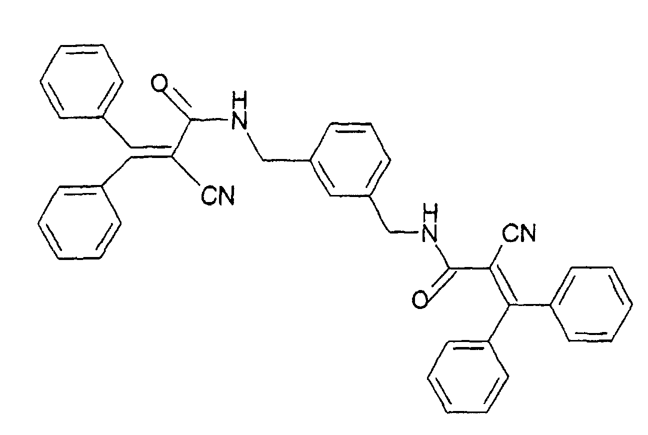

35. Emulsion selon l'une quelconque des revendications 1 à 14, où les filtres UV insolubles sont des sels de métaux polyvalents de filtres UV organiques sulfoniques ou carboxyliques.

36. Emulsion selon l'une quelconque des revendications 1 à 14, où les filtres UV insolubles sont choisis parmi les sels de métaux polyvalents de dérivés sulfonés de benzylidène camphre ; les sels de métaux polyvalents de dérivés sulfonés de benzimidazole ; les sels de métaux polyvalents de dérivés d'acide cinnamique.

37. Emulsion selon l'une quelconque des revendications 1 à 14, où les filtres UV insolubles sont des complexes de métaux polyvalents ou d'ammonium ou d'ammonium substitué de filtres organiques UV-A et/ou UV-B.

38. Emulsion selon la revendication 37, où le ou les filtres UV insolubles sous forme insoluble micronisée a une taille moyenne de particule allant de 0,02 à 1,5 µm.

39. Emulsion selon la revendication 38, où le ou les filtres UV insolubles sous forme insoluble micronisée a une taille moyenne de particule allant de 0,03 à 1,0 µm.

40. Emulsion selon l'une quelconque des revendications 1 à 39, **caractérisée par le fait que** le ou les filtres UV insolubles sous forme micronisée sont susceptibles d'être obtenus par un procédé de broyage du filtre organique insoluble sous forme de particules de taille grossière en présence d'un tensio-actif.

41. Emulsion selon la revendication 40, où le tensio-actif est choisi parmi les alkylpolyglucosides de structure CₙH₂ₙ₊₁O(C₆H₁₀O₅)ₓH dans laquelle n est un entier de 8 à 16 et x est le degré moyen de polymérisation de l'unité (C₆H₁₀O₅) et varie de 1,4 à 1,6.

42. Emulsion selon l'une quelconque des revendications 40 à 41, **caractérisée par le fait que** le tensio-actif est utilisé à une concentration allant de 1 à 50% en poids par rapport au filtre insoluble dans sa forme micronisée.

43. Composition cosmétique ou dermatologique, destinée à la photoprotection de la peau et/ou des cheveux, **caractérisée par le fait qu'**elle comprend dans un support cosmétiquement acceptable une émulsion telle que définie dans l'une quelconque des revendications 1 à 42.

44. Composition selon la revendication 43, **caractérisée par le fait qu'**elle comprend en outre un ou plusieurs filtres organiques complémentaires actifs dans l'UV-A et/ou UV-B, solubles dans au moins l'une des phases de la composition.

45. Composition selon la revendication 44, **caractérisée par le fait que** lesdits filtres organiques complémentaires sont choisis parmi les dérivés cinnamiques ; les dérivés salicyliques, les dérivés du camphre; les dérivés de triazine ; les dérivés de dibenzoylméthane; les dérivés de la benzophénone ; les dimères dérivés d'α-alkylstyrène ; les dérivés de β,β'-diphénylacrylate, les dérivés de benzimidazole ; les dérivés bis-benzoazolyle; les dérivés de l'acide p-aminobenzoïque ; les polymères filtres et silicones filtres.

46. Composition selon l'une quelconque des revendications 43 à 45, **caractérisée par le fait qu'**elle comprend en outre des pigments ou des nanopigments d'oxydes métalliques, enrobés ou non.

47. Composition selon la revendication 46, **caractérisée par le fait que** lesdits pigments ou nanopigments sont choisis parmi les oxydes de titane, de zinc, de fer, de zirconium, de cérium et leurs mélanges, enrobés ou non.

48. Composition selon l'une quelconque des revendications 43 à 47, **caractérisée par le fait qu'**elle comprend en outre au moins un agent de bronzage et/ou de brunissage artificiel de la peau.

49. Composition selon l'une quelconque des revendications 43 à 48, **caractérisée par le fait qu'**elle comprend en outre au moins un adjuvant choisi parmi les corps gras, les solvants organiques, les épaississants, les adoucissants, les opacifiants, les stabilisants, les émollients, les agents anti-mousse, les agents hydratants, les parfums, les conservateurs, les polymères, les séquestrants, les propulseurs, les agents alcalinisants ou acidifiants.

50. Composition selon l'une quelconque des revendications 43 à 49, **caractérisée par le fait qu'**il s'agit d'une composition protectrice de l'épiderme humain ou d'une composition antisolaire et qu'elle se présente sous forme d'une dispersion vésiculaire non ionique, d'une crème, d'un lait, d'un gel, d'un gel crème, d'une suspension, d'une dispersion, d'une poudre, d'un bâtonnet solide, d'une mousse ou d'un spray.

51. Composition selon l'une quelconque des revendications 43 à 49, **caractérisée par le fait qu'**il s'agit d'une composition de maquillage des cils, des sourcils ou-de la peau et qu'elle se présente sous forme solide ou pâteuse, anhydre ou aqueuse, d'une émulsion, d'une suspension ou d'une dispersion.

52. Composition selon l'une quelconque des revendications 43 à 49, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

53. Utilisation de l'émulsion définie dans l'une quelconque des revendications 1 à 42 pour la fabrication de compositions cosmétiques pour la protection de la peau et/ou des cheveux contre le rayonnement ultraviolet, en particulier le rayonnement solaire.

## Patentansprüche

1. Kosmetische oder dermatologische Wasser-in-Öl-Emulsion, **dadurch gekennzeichnet, daß** sie enthält:
(a) mindestens eine wässerige Phase und
(b) mindestens eine Fettphase;
(c) mindestens ein Lichtschutzsystem, das befähigt ist, die UV-Strahlung zu filtern, und das mindestens ein in der Emulsion unlösliches organisches UV-Filter in mikronisierter Form enthält, dessen mittlere Größe der Partikel im Bereich von 0,01 bis 2 µm liegt;
(d) mindestens ein nicht-filterndes organomodifiziertes Silicon, das alkoxylierte Gruppen enthält;
mit der Maßgabe, daß das unlösliche organische UV-Filter von 2,4,6-Tris[p-(2'-ethylhexyl-1'-oxycarbonyl)-anilino]-1,3,5-triazin in mikronisierter unlöslicher Form und der Verbindung der folgenden Struktur 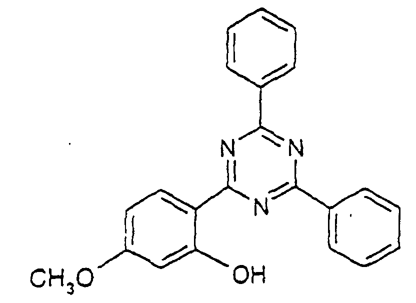 in mikronisierter unlöslicher Form verschieden ist.

2. Emulsion nach Anspruch 1, wobei das nicht-filternde organomodifizierte Silicon als Öl, Wachs, Harz oder Gummi vorliegt, die wasserlöslich oder in Wasser unlöslich sind.

3. Emulsion nach Anspruch 1, wobei das nicht-filternde organomodifizierte Silicon unter den Polyorganosiloxanen ausgewählt ist, die ethoxylierte und/oder propoxylierte Gruppen enthalten.

4. Emulsion nach einem der Ansprüche 1 bis 3, wobei das nicht-filternde organomodifizierte Silicon der folgenden. Formel entspricht: 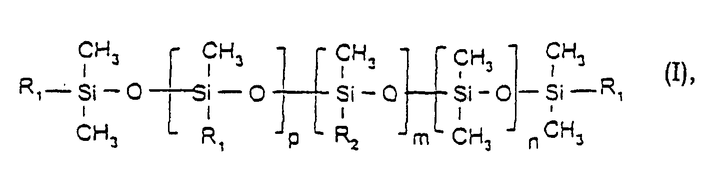 worin
- die Gruppen R₁, die identisch oder voneinander verschieden sind, eine geradkettige oder verzweigte C₁₋₃₀-Alkylgruppe oder Phenyl bedeuten;
- die Gruppen R₂, die identisch oder voneinander verschieden sind, eine Gruppe -C_{c}H_{2c}-(-O-C₂H₄)ₐ-(-O-C₃H₆)_{b}-(O-C₄H₈)_{d}-R₃ bedeuten;
- die Gruppen R₃, die identisch oder voneinander verschieden sind, unter Wasserstoff, Hydroxy, einer geradkettigen oder verzweigten Alkylgruppe mit 1 bis 12 Kohlenstoffatomen, einer geradkettigen oder verzweigten Alkoxygruppe mit 1 bis 6 Kohlenstoffatomen, einer geradkettigen oder verzweigten Acyloxygruppe mit 2 bis 12 Kohlenstoffatomen, NHCH₂CH₂COOM, einer Aminoalkylgruppe, die gegebenenfalls am Amin substituiert ist, C₁₋₃₀-Carboxyacyl, einer gegebenenfalls substituierten Phosphonogruppe, -O-CO-(CH₂)_{d}-CO₂M, -NHCO(CH₂)_{d}OH oder -NH₃Y ausgewählt sind;
- die Gruppen M, die identisch oder voneinander verschieden sind, Wasserstoff, Na, K, Li, NH₄ oder ein organisches Amin bedeuten und Y ein einwertiges, anorganisches oder organisches Anion ist, beispielsweise ein Halogenid (Chlorid, Bromid), Sulfat oder Carboxylat (Acetat, Lactat, Citrat);
- a im Bereich von 0 bis 100 liegt;
- b im Bereich von 0 bis 50 liegt;
- c im Bereich von 0 bis 5 liegt;
- a + b mindestens 1 ist;
- d im Bereich von 0 bis 10 liegt;
- m im Bereich von 0 bis 20 liegt;
- n im Bereich von 0 bis 500 liegt;
- p im Bereich von 0 bis 50 liegt.

5. Emulsion nach Anspruch 4, wobei das nicht-filternde organomodifizierte Silicon der Formel I unter den Verbindungen ausgewählt ist, worin die Gruppen R₁ alle Methyl bedeuten und die Gruppe R₃ Hydroxy ist.

6. Emulsion nach Anspruch 5, wobei das nicht-filternde organomodifizierte Silicon der Formel I das ethoxylierte und propoxylierte Polydimethyl/methylsiloxan (EO/PO 18/18) ist, worin p+n 396 und m 4 ist (CTFA-Bezeichnung: Cyclometicon 90 %, Dimeticoncopolyol 10 %).

7. Emulsion nach Anspruch 4, wobei das nicht-filternde orgariomodifizierte Silicon der Formel I unter den Verbindungen ausgewählt ist, worin die Gruppen R₁ Methylgruppen und C₂₋₃₀-Alkylgruppen bedeuten und die Gruppe R₃ Hydroxy ist.

8. Emulsion nach Anspruch 7, wobei das nicht-filternde organomodifizierte Silicon der Formel I das ethoxylierte und propoxylierte Polymethyllauryl/methylsiloxan (EO/PO 18/18) ist, worin p+n 35 und m 3 ist (CTFA-Bezeichnung: Laurylmeticoncopolyol 91 %, Isostearylalkohol 9 %).

9. Emulsion nach Anspruch 7, wobei das nicht-filternde organomodifizierte Silicon der Formel I das ethoxylierte und propoxylierte Polymethylcetyl/methylsiloxan ist, worin p 20 bis 25, m 5 und n 75 ist (CTFA-Bezeichnung: Cetyldimeticoncopolyol).

10. Emulsion nach einem der Ansprüche 1 bis 3, wobei das nicht-filternde orgariomodifizierte Silicon ein in α-ω-Stellung substituiertes, alkoxyliertes Silicon von geradkettiger Struktur ist, das an beiden Enden der Hauptkette mit alkoxylierten Gruppen substituiert ist, die an die Siliciumatome über eine Kohlenwasserstoffgruppe gebunden sind.

11. Emulsion nach Anspruch 10, wobei das nicht-filternde organomodifizierte Silicon ein Silicon der folgenden allgemeinen Formel II ist: 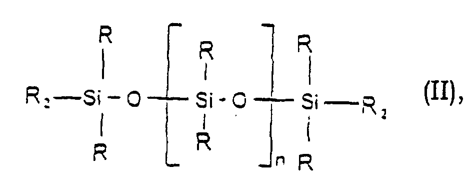 worin n im Bereich von 1 bis 500 liegt; die Gruppen R₂, die identisch oder voneinander verschieden sind, eine Gruppe -C_{c}H_{2c}-(-O-C₂H₄)ₐ-(-O-C₃H₆)_{b}-(O-C₄H₈)_{d}-R₃ bedeuten, wobei a, b, c und R₃ die für Formel I angegebenen Bedeutungen aufweisen, und die Gruppen R, die identisch oder voneinander verschieden sind, eine geradkettige oder verzweigte C₁₋₃₀-Alkylgruppe oder Phenyl bedeuten.

12. Emulsion nach Anspruch 11, wobei alle Gruppen R Methylgruppen sind; c im Bereich von 2 bis 4 liegt, a im Bereich von 3 bis 100 liegt, b im Bereich von 1 bis 50 liegt, d 0 ist, R₃ eine Hydroxygruppe bedeutet und n im Bereich von 50 bis 200 liegt.

13. Emulsion nach Anspruch 11 oder 12, wobei das Gewichtsverhältnis der Einheiten C₂H₄O und der Einheiten C₃H₆O im Bereich von 100:10 bis 20:80 liegt.

14. Emulsion nach einem der Ansprüche 1 bis 13, wobei das nicht-filternde organomodifizierte Silicon oder die nicht-filternden organomodifizierten Silicone in Mengenanteilen von 0,1 bis 20 %, bezogen auf das Gesamtgewicht der Emulsion, und vorzugsweise in einer Menge von 0,5 bis 10 % vorliegen.

15. Emulsion nach einem der Ansprüche 1 bis 14, wobei das oder die mikronisierten, unlöslichen, organischen UV-Filter unter den organischen UV-Filtern vom Oxanilidtyp, Triazintyp, Triazoltyp, Amidvinyltyp oder Cinnamamidtyp ausgewählt sind.

16. Emulsion nach Anspruch 15, wobei die UV-Filter vom Oxanilidtyp der folgenden Struktur entsprechen: 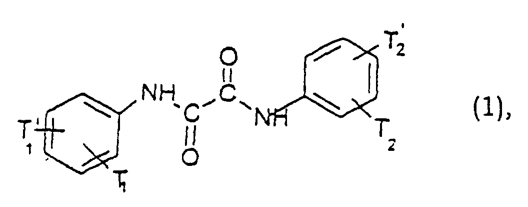 worin T₁, T'₁, T₂ und T'₂, die identisch oder voneinander verschieden sind, eine C₁₋₈-Alkylgruppe oder eine C₁₋₈-Alkoxygruppe bedeuten.

17. Emulsion nach Anspruch 15, wobei die UV-Filter vom Triazirityp unter den Verbindungen der folgenden allgemeinen Formel ausgewählt sind: 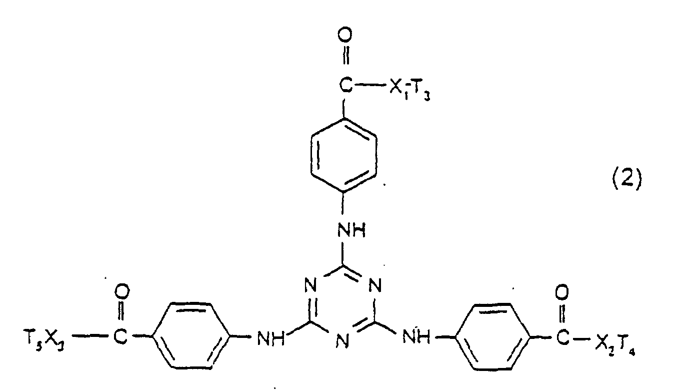 worin:
- die Gruppen X₁, X₂ und X₃, die identisch oder voneinander verschieden sind, Sauerstoff oder eine Gruppe -NZ bedeuten;
- die Gruppen Z, die identisch oder voneinander verschieden sind, Wasserstoff, eine geradkettige oder verzweigte C₁₋₁₈-Alkylgruppe oder eine C₅₋₁₂-Cycloalkyigruppe, die mit einer oder mehreren C₁₋₄-Alkylgruppen substituiert sein kann, bedeuten;
- die Gruppen T₃, T₄ und Ts, die identisch oder voneinander verschieden sind, ausgewählt sind unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer polyethoxylierten Gruppe mit 1 bis 6 Ethylenoxideinheiten, deren OH-Endgruppe methyliert ist; einer Gruppe der folgenden Formeln 3, 4 oder 5: 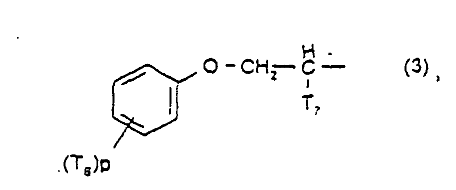 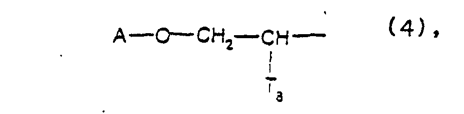 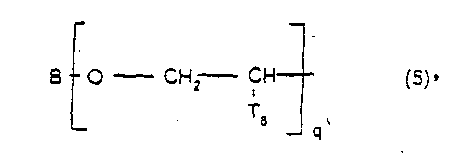
worin bedeuten:
- T₆ Wasserstoff oder Methyl;
- T₇ C₁₋₉-Alkyl;
- p 0 oder eine ganze Zahl von 1 bis 3;
- q eine ganze Zahl von 1 bis 10;
- A eine C₄₋₈-Alkylgruppe oder eine C₅₋₈-Cycloalkylgruppe;
- B ist ausgewählt unter: einer geradkettigen oder verzweigten C₁₋₈-Alkylgruppe; einer C₅₋₈-Cycloalkylgruppe; einer gegebenenfalls mit einer oder mehreren C₁₋₄-Alkylgruppen substituierten Arylgruppe;
- T₈ Wasserstoff oder Methyl.

18. Emulsion nach Anspruch 17, wobei das UV-Filter vom Triazintyp der folgenden Formel entspricht: 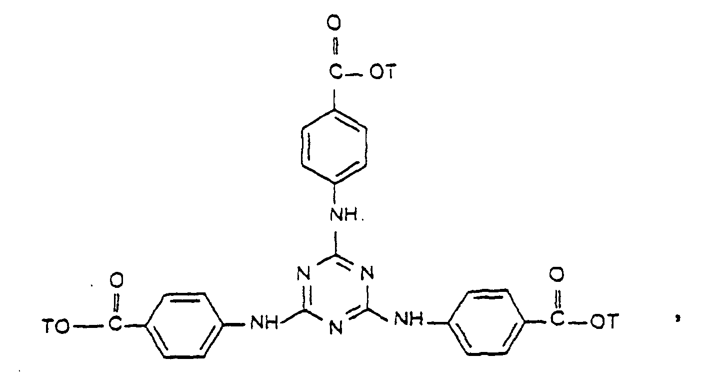 worin T eine 2-Ethylhexylgruppe bedeutet.

19. Emulsion nach Anspruch 17, wobei das UV-Filter vom Triazintyp der folgenden Formel entspricht: 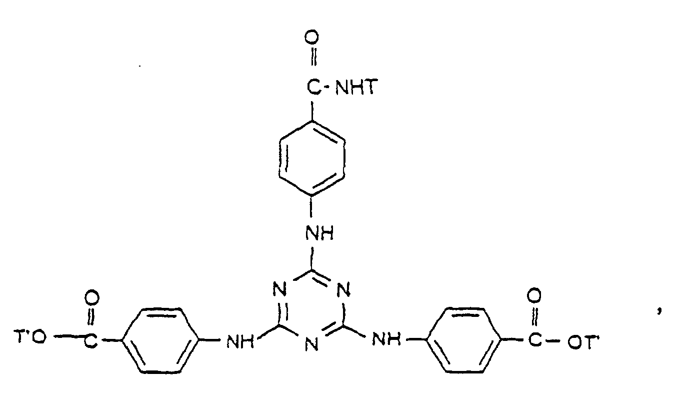 worin T' eine 2-Ethylhexylgruppe und T eine t-Butylgruppe bedeutet.

20. Emulsion nach Anspruch 15, wobei die UV-Filter vom Triazintyp unter den unlöslichen s-Triazinderivaten ausgewählt sind, die Benzalmalonat- und/oder Phenylcyanoacrylatgruppen enthalten.

21. Emulsion nach Anspruch 20, wobei die UV-Filter vom Triazintyp unter den folgenden Verbindungen ausgewählt sind:
- 2,4,6-Tris(diethyl-4'-aminobenzalmalonat)-s-triazin,
- 2,4,6-Tris(diisopropyl-4'-aminobenzalmalonat)-s-triazin,
- 2,4,6-Tris(dimethyl-4'-aminobenzalmalonat)-s-triazin,
- 2,4,6-Tris(ethyl-a-cyano-4-aminocinnamat)-s-triazin.

22. Emulsion nach Anspruch 15, wobei die UV-Filter vom Triazintyp unter den Verbindungen der folgenden Formeln ausgewählt sind: 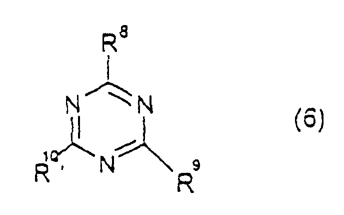 worin die Gruppen R⁸, R⁹ und R¹⁰ unabhängig voneinander Phenyl, Phenoxy oder Pyrrolo bedeuten, wobei die Phenyl-, Phenoxy- oder Pyrrologruppen gegebenenfalls mit einem, zwei oder drei der folgenden Substituenten substituiert sind: OH, C₁₋₁₈-Alkyl oder C₁₋₁₈-Alkoxy, C₁₋₁₈-Carboxyalkyl, C₅₋₈-Cycloalkyl, Methylidencampher, -(CH=CH)ₙ(CO)-OR⁴, wobei R⁴ entweder C₁₋₁₈-Alkyl oder Cinnamyl bedeutet und n 0 oder 1 ist.

23. Emulsion nach Anspruch 15, wobei die UV-Filter vom Triazintyp unter den unlöslichen s-Triazinderivaten ausgewählt sind, die Benzotriazol- und/oder Benzothiazolgruppen enthalten.

24. Emulsion nach Anspruch 23, wobei die unlöslichen UV-Filter vom Triazintyp ausgewählt sind unter:
- 2,4,6-Tris[(3'-benzotriazol-2-yl-2'-hydroxy-5'-methyl)-phenylamino]-s-triazin,
- 2,4,6-Tris[(3'-benzotriazol-2-yl-2'-hydroxy-5'-ter-octyl)-phenylamino]-s-triazin.

25. Emulsion nach Anspruch 15, wobei die organischen UV-Filter vom Triazoltyp der folgenden Formel 7 entsprechen: 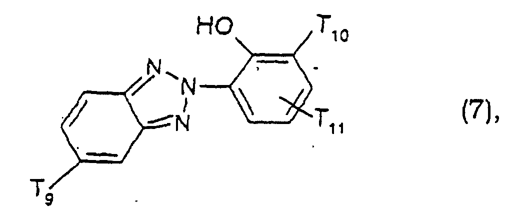 wobei T₉ ein Wasserstoffatom oder eine C₁₋₁₈-Alkylgruppe bedeutet und T₁₀ und T₁₁, die identisch oder voneinander verschieden sind, eine gegebenenfalls mit einer Phenylgruppe substituierte C₁₋₁₈-Alkylgruppe bedeuten.

26. Emulsion nach Anspruch 25, wobei die Verbindung der Formel 7 unter den folgenden Verbindungen ausgewählt ist,

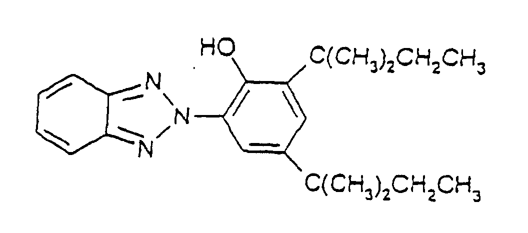

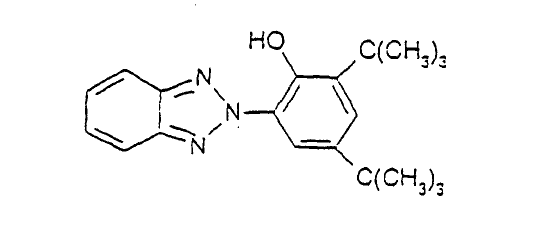

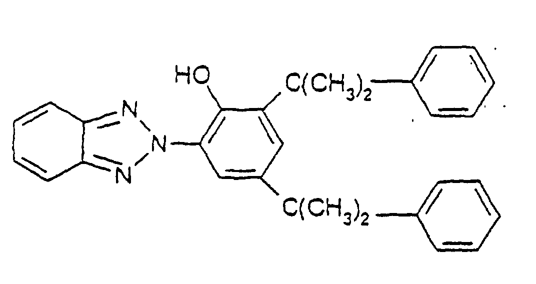

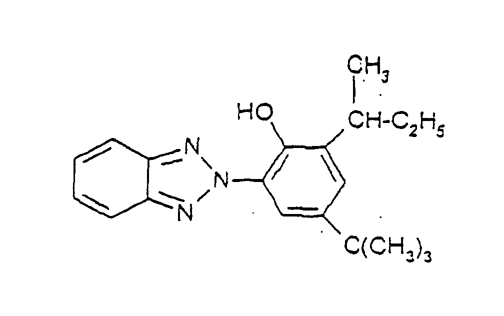

27. Emulsion nach Anspruch 15, wobei das unlösliche UV-Filter in mikronisierter Form das [2,4'-Dihydroxy-3-(2H-benzotriazol-2-yl)-5-(1,1,3,3-tetramethylbutyl)-2'-n-octoxy-5'-benzoyl]diphenylmethan der folgenden Struktur ist:

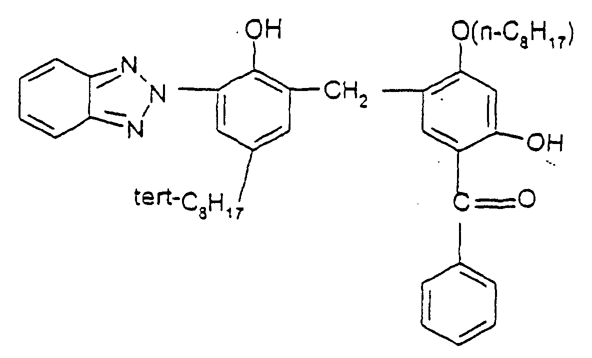

28. Emulsion nach Anspruch 15, wobei die organischen UV-Filter vom Triazoltyp unter den Methylen-bis(hydroxyphenylbenzotriazol)-Derivaten der folgenden Struktur ausgewählt sind: 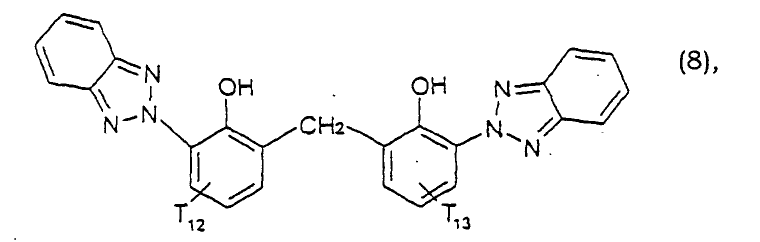 worin die Gruppen T₁₂ und T₁₃, die identisch oder voneinander verschieden sind, eine C₁₋₁₈-Alkylgruppe, die mit einer oder mehreren Gruppen substituiert sein kann, die unter den C₁₋₄-Alkylgruppen oder C₅₋₁₂-Cycloalkylgruppen ausgewählt sind, oder eine Arylgruppe bedeuten.

29. Emulsion nach Anspruch 28, wobei die Verbindung der Formel 8 unter den Verbindungen der folgenden Struktur ausgewählt ist:

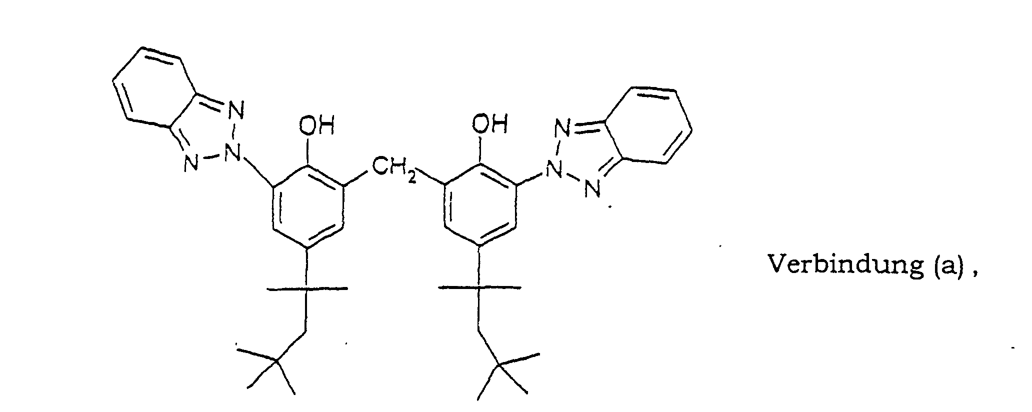

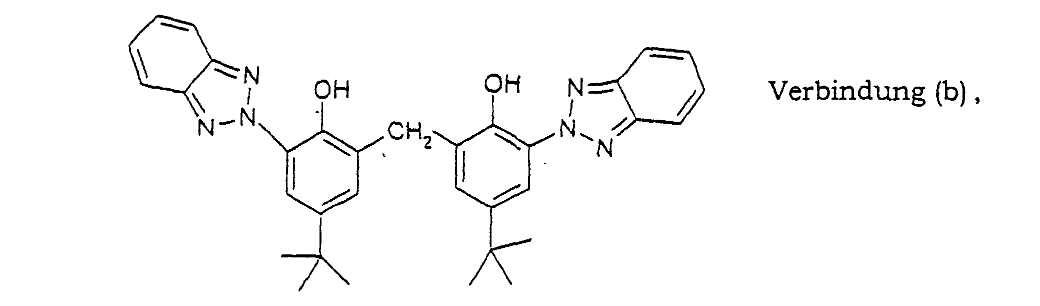

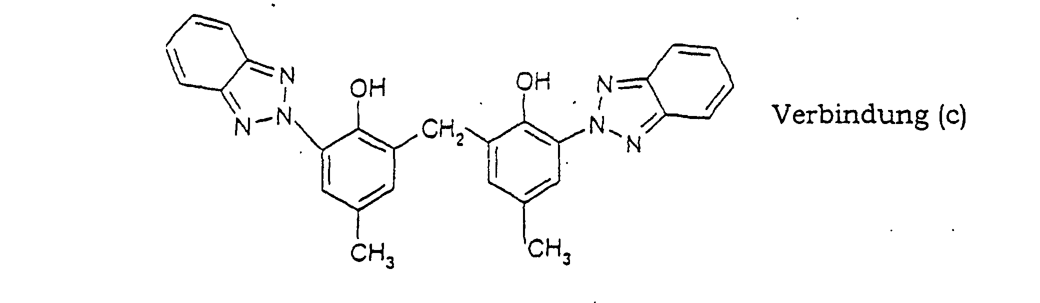

30. Emulsion nach Anspruch 15, wobei die organischen Filter vom Amidvinyltyp der folgenden Formel entsprechen:
T₁₄-(Y)ᵣ-C(=O)-C(T₁₅)=C(T₁₆)-N(T₁₇)(T₁₈) (9),
worin T₁₄ eine C₁₋₁₈-Alkylgruppe und vorzugsweise eine C₁₋₅-Alkylgruppe, eine Phenylgruppe, die gegebenenfalls mit einer, zwei oder drei Gruppen substituiert ist, die ausgewählt sind unter: OH, C₁₋₁₈-Alkyl oder C₁₋₈-Alkoxy, oder -C(=O)-OT₁₉ bedeutet, wobei T₁₉ eine C₁₋₁₈-Alkylgruppe ist; die Gruppen T₁₅, T₁₆, T₁₇ und T₁₈, die identisch oder voneinander verschieden sind, eine C₁₋₁₈-Alkylgruppe und vorzugsweise eine C₁₋₅-Alkylgruppe oder ein Wasserstoffatom bedeuten; Y N oder O ist und r 0 oder 1 bedeutet.

31. Emulsion nach Anspruch 30, wobei die Verbindungen der Formel 9 ausgewählt sind unter:
- 4-Octylamino-3-penten-2-on;
- Ethyl-3-octylamino-2-butenoat;
- 3-Octylamino-1-phenyl-2-buten-1-on;
- 3-Dodecylamino-1-phenyl-2-buten-1-on.

32. Emulsion nach Anspruch 15, wobei die organischen Filter vom Cinnamamidtyp der folgenden Formel entsprechen: 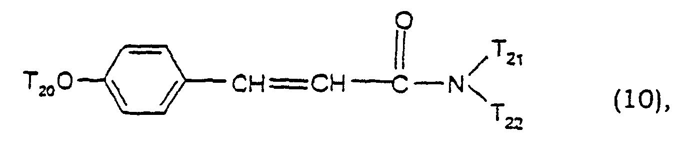 worin OT₂₀ eine Hydroxygruppe oder eine C₁₋₄-Alkoxygruppe ist, vorzugsweise Methoxy oder Ethoxy; T₂₁ Wasserstoff, C₁₋₄-Alkyl und vorzugsweise Methyl oder Ethyl bedeutet; T₂₂ eine Gruppe -(CONH)ₛ-phenyl, wobei s 0 oder 1 bedeutet und die Phenylgruppe mit einer, zwei oder drei Gruppen substituiert sein kann, die unter OH, C₁₋₁₈-Alkyl oder C₁₋₈-Alkoxy ausgewählt sind oder -C(=O)-OT₂₃ bedeutet, wobei T₂₃ eine C₁₋₁₈-Alkylgruppe und noch bevorzugter eine Phenylgruppe, 4-Methoxyphenyl oder Phenylaminocarbonyl ist.

33. Emulsion nach Anspruch 15, wobei das unlösliche Filter ein Cinnamid-Dimer ist.

34. Emulsion nach Anspruch 33, wobei das unlösliche UV-Filter die Verbindung der folgenden Struktur ist:

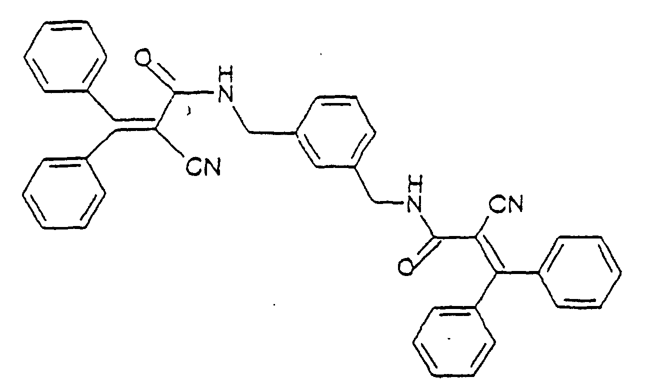

35. Emulsion nach einem der Ansprüche 1 bis 14, wobei die unlöslichen UV-Filter mehrwertige Metallsalze von organischen Sulfonfiltern oder Carboxyfiltern sind.

36. Emulsion nach einem der Ansprüche 1 bis 14, wobei die unlöslichen UV-Filter unter den mehrwertigen Metallsalzen von sulfonierten Benzylidencampherderivaten; mehrwertigen Metallsalzen von sulfonierten Benzimidazolderivaten; und mehrwertigen Metallsalzen von Zimtsäurederivaten ausgewählt sind.

37. Emulsion nach einem der Ansprüche 1 bis 14, wobei es sich bei den unlöslichen UV-Filtern um Komplexe von mehrwertigen Metallen oder Ammonium oder substituiertem Ammonium und organischen UV-A- und/oder UV-B-Filtern handelt.

38. Emulsion nach Anspruch 37, wobei das oder die unlösliche(n) UV-Filter, die in unlöslicher, mikronisierter Form vorliegen, eine mittlere Größe der Partikel im Bereich von 0,02 bis 1,5 µm aufweisen.

39. Emulsion nach Anspruch 38, wobei das oder die unlösliche(n) UV-Filter, die in unlöslicher, mikronisierter Form vorliegen, eine mittlere Größe der Partikel im Bereich von 0,03 bis 1,0 µm aufweisen.

40. Emulsion nach einem der Ansprüche 1 bis 39, **dadurch gekennzeichnet, daß** das oder die unlösliche(n) UV-Filter in mikronisierter Form durch Zerkleinern eines unlöslichen organischen Filters, das in Form von groben Partikel vorliegt, in Gegenwart eines grenzflächenaktiven Stoffes hergestellt werden kann.

41. Emulsion nach Anspruch 40, wobei der grenzflächenaktive Stoff unter den Alkylpolyglucosiden der Struktur CₙH₂ₙ₊₁O(C₆H₁₀O₅)ₓH ausgewählt ist, worin n eine ganze Zahl von 8 bis 16 bedeutet und x der mittlere Polymerisationsgrad der Einheit (C₆H₁₀O₅) ist und im Bereich von 1,4 bis 1,6 liegt.

42. Emulsion nach einem der Ansprüche 40 bis 41, **dadurch gekennzeichnet, daß** der grenzflächenaktive Stoff in einer Konzentration von 1 bis 50 Gew.-%, bezogen auf das in mikronisierter Form vorliegende unlösliche Filter verwendet wird.

43. Kosmetische oder dermatologische Zusammensetzung, die zum Lichtschutz der Haut und/oder der Haare vorgesehen ist, **dadurch gekennzeichnet, daß** sie in einem kosmetisch akzeptablen Träger eine Emulsion nach einem der Ansprüche 1 bis 42 enthält.

44. Zusammensetzung nach Anspruch 43, **dadurch gekennzeichnet, daß** sie ferner ein oder mehrere zusätzliche, im UV-A- und/oder UV-B-Bereich wirksame organische Filter enthält, die in mindestens einer der Phasen der Zusammensetzung löslich sind.

45. Zusammensetzung nach Anspruch 44, **dadurch gekennzeichnet, daß** die zusätzlichen organischen Filter ausgewählt sind unter: Zimtsäurederivaten, Salicylsäurederivaten, Campherderivaten, Triazinderivaten, Dibenzoylmethanderivaten, Benzophenonderivaten, von α-Alkylstyrol abgeleiteten Dimeren, β,β'-Diphenylacrylatderivaten, Benzimidazolderivaten, Bisbenzoazolylderivaten, p-Aminobenzolsäurederivaten, polymeren Filtern und Siliconfiltern.

46. Zusammensetzung nach einem der Ansprüche 43 bis 45, **dadurch gekennzeichnet, daß** sie außerdem Pigmente oder Nanopigmente von Metalloxiden enthält, die gegebenenfalls umhüllt sind.

47. Zusammensetzung nach Anspruch 46, **dadurch gekennzeichnet, daß** die Pigmente oder Nanopigmente unter den Oxiden von Titan, Zink, Eisen, Zirconium, Cer und deren Gemischen ausgewählt sind, wobei die Pigmente oder Nanopigmente gegebenenfalls umhüllt sind.

48. Zusammensetzung nach einem der Ansprüche 43 bis 47, **dadurch gekennzeichnet, daß** sie außerdem mindestens ein Mittel zur Bräunung und/oder künstlichen Braunfärbung der Haut enthält.

49. Zusammensetzung nach einem der Ansprüche 43 bis 48, **dadurch gekennzeichnet, daß** sie ferner mindestens einen Zusatzstoff enthält, der unter den Fettsubstanzen, organischen Lösungsmitteln, Verdickungsmitteln, Mitteln für die Geschmeidigkeit, Trübungsmitteln, Stabilisierungsmitteln, Emollientien, Antischaummitteln, Hydratisierungsmitteln, Parfums, Konservierungsmitteln, Polymeren, Maskierungsmitteln, Treibmitteln, Alkalisierungsmitteln oder Ansäuerungsmitteln ausgewählt ist.

50. Zusammensetzung nach einem der Ansprüche 43 bis 49, **dadurch gekennzeichnet, daß** es sich um eine Zusammensetzung zum Schutz der menschlichen Epidermis oder ein Sonnenschutzmittel handelt und dadurch, daß sie als nichtionische Vesikeldispersion, Creme, Milch, Gel, Gelcreme, Suspension, Dispersion, Pulver, fester Stift, Schaum oder Spray vorliegt.

51. Zusammensetzung nach einem der Ansprüche 43 bis 49, **dadurch gekennzeichnet, daß** es sich um eine Zusammensetzung zum Schminken der Wimpern, Augenbrauen oder der Haut handelt und dadurch, daß sie in fester oder pastöser, wasserfreier oder wässeriger Form, als Emulsion, Suspension oder Dispersion vorliegt.

52. Zusammensetzung nach einem der Ansprüche 43 bis 49, **dadurch gekennzeichnet, daß** es sich um eine Zusammensetzung handelt, die zum Schutz der Haare gegen UV-Strahlung vorgesehen ist, und dadurch, daß sie als Haarwaschmittel, Lotion, Gel, Emulsion oder nichtionische Vesikeldispersion vorliegt.

53. Verwendung einer Emulsion nach einem der Ansprüche 1 bis 42 zur Herstellung von kosmetischen Zusammensetzungen zum Schutz der Haut und/oder der Haare gegen UV-Strahlung und insbesondere gegen Sonnenlicht.

## Claims

1. Cosmetic or dermatological water-in-oil emulsion, **characterized in that** it comprises:
(a) at least one aqueous phase and
(b) at least one fatty phase;
(c) at least one photoprotective system capable of screening out UV rays, containing at least one organic UV-screening agent insoluble in the emulsion, in micronized form, in which the mean size of the particles varies from 0.01 to 2 µm;
(d) at least one non-screening organomodified silicone comprising oxyalkylenated groups;
with the proviso that the said insoluble organic UV-screening agent is different from 2,4,6-tris[p-(2'-ethylhexyl-1'-oxycarbonyl)anilino]-1,3,5-triazine in micronized insoluble form and the compound having the structure 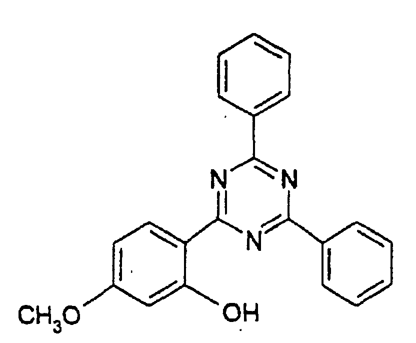 in micronized insoluble form.

2. Emulsion according to Claim 1, where the non-screening organomodified silicone is provided in the form of an oil, a wax, a resin or a gum, which is water-soluble or insoluble in water.

3. Emulsion according to Claim 1, where the non-screening organomodified silicone is chosen from polyorganosiloxanes comprising oxyethylenated and/or oxypropylenated groups.

4. Emulsion according to any one of Claims 1 to 3, where the non-screening organomodified silicone corresponds to the following formula: 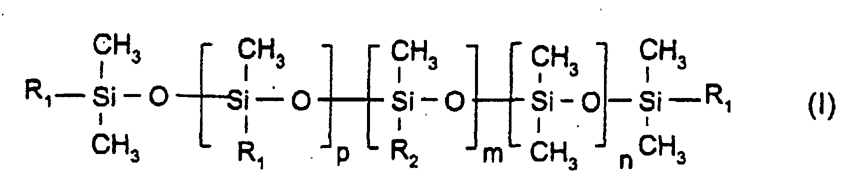
- the radicals R₁, identical or different, represent a linear or branched C₁-C₃₀ alkyl radical or a phenyl radical,
- the radicals R₂, identical or different, denote a group
-C_{c}H_{2c}- (-O-C₂H₄)ₐ- (-O-C₃H₆)_{b}- (O-C₄H₈)_{d}-R₃,
- the radicals R₃, identical or different, are chosen from a hydrogen atom, a hydroxyl radical, a linear or branched alkyl radical having from 1 to 12 carbon atoms, a linear or branched alkoxy radical having from 1 to 6 carbon atoms, a linear or branched acyloxy radical having from 2 to 12 carbon atoms, a radical NHCH₂CH₂COOM, an aminoalkyl radical optionally substituted on the amine, a C₁-C₃₀ carboxyacyl radical, an optionally substituted phosphono group, a group -O-CO-(CH₂)_{d}-CO₂M, -NHCO(CH₂)_{d}OR or -NH₃Y,
- M, identical or different, denotes a hydrogen atom, Na, K, Li, NH₄ or an organic amine, Y represents a monovalent inorganic or organic anion such as halide (chloride, bromide), sulphate, carboxylate (acetate, lactate, citrate),
- a varies from 0 to 100,
- b varies from 0 to 50,
- c varies from 0 to 5,
- a + b is greater than or equal to 1,
- d varies from 0 to 10,
- m varies from 0 to 20,
- n varies from 0 to 500,
- p varies from 0 to 50.

5. Emulsion according to Claim 4, where the non-screening organomodified silicone of formula (I) is chosen from those for which the radicals R₁ all represent methyl radicals and the radical R₃ represents an hydroxyl radical.

6. Emulsion according to Claim 5, where the non-screening organomodified silicone of formula (I) is oxyethylenated oxypropylenated polydimethyl/methyl siloxane (EO/PO 18/18) for which p+n is 396 and m is 4, (CTFA name: cyclomethicone 90% dimethicone copolyol 10%).

7. Emulsion according to Claim 4, where the non-screening organomodified silicone of formula (I) is chosen from those for which the radicals R₁ represent methyl radicals and C₂-C₃₀ alkyl radicals and the radical R₃ represents a hydroxyl radical.

8. Emulsion according to Claim 7, where the non-screening organomodified silicone of formula (I) is oxyethylenated oxypropylenated polymethyllauryl/methyl siloxane (EO/PO 18/18) for which p+n is 35 and m is 3, (CTFA name: laurylmethicone copolyol 91%, isostearyl alcohol 9%).

9. Emulsion according to Claim 7, where the non-screening organomodified silicone of formula (I) is oxyethylenated oxypropylenated polymethylcetyl/methyl siloxane, for which p is 20 to 25 and m is 5 and n is 75 (CTFA name: cetyldimethicone copolyol).

10. Emulsion according to any one of Claims 1 to 3, where the non-screening organomodified silicone is an α-ω-substituted oxyalkylenated silicone having a linear structure, substituted at the two ends of the principal chain with oxyalkylenated groups linked to the Si atoms via a hydrocarbon group.

11. Emulsion according to Claim 10, where the non-screening organomodified silicone is a silicone corresponding to the following general formula (II): 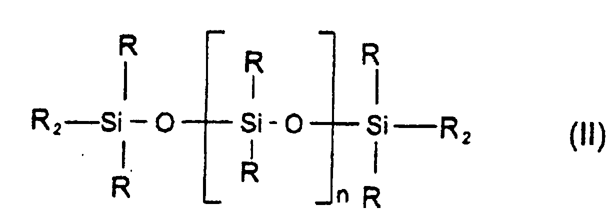 in which n varies from 1 to 500; the radicals R₂, identical or different, denote à group -C_{c}H_{2c}- (-O-C₂H₄)ₐ- (-O-C₃H₆)_{b}- (O-C₄H₈)_{d}-R₃ where a, b, c and R₃ have the same definitions indicated in formula (I) and the radicals R, identical or different, represent a linear or branched C₁-C₃₀ alkyl radical or a phenyl radical.

12. Emulsion according to Claim 11, where all the radicals R are methyl radicals; c ranges from 2 to 4, a ranges from 3 to 100, b ranges from 1 to 50, d equals 0, R₃ is a hydroxyl radical, n ranges from 50 to 200.

13. Emulsion according to Claim 11 or 12, where the weight ratio of the C₂H₄O units relative to the C₃H₆O units ranges from 100:10 to 20:80.

14. Emulsion according to any one of Claims 1 to 13, in which the non-screening organomodified silicone(s) are present in quantities ranging from 0.1 to 20% relative to the total weight of the emulsion, preferably in a quantity ranging from 0.5 to 10%.

15. Emulsion according to any one of Claims 1 to 14, where the micronized insoluble organic UV-screening agent(s) are chosen from the organic UV-screening agents of the oxanilide type, of the triazine type, of the triazole type, of the vinylamide type or of the cinnamamide type.

16. Emulsion according to Claim 15, where the UV-screening agents of the oxanilide type correspond to the structure: 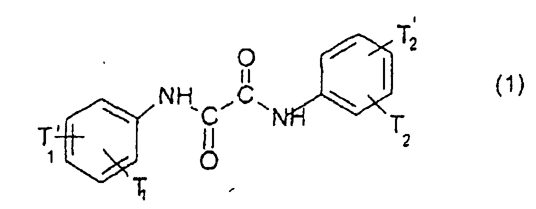 in which T₁, T'₁, T₂ and T'₂, which are identical or different, denote a C₁-C₈ alkyl radical or a C₁-C₈ alkoxy radical.

17. Emulsion according to Claim 15, where the UV-screening agents of the triazine type are chosen from those corresponding to the following general formula: 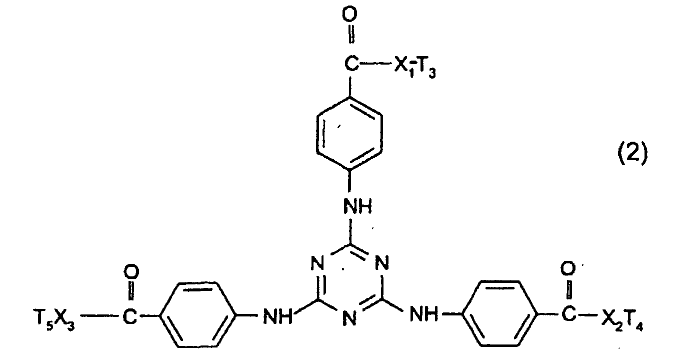 in which:
- X₁, X₂ and X₃, which are identical or different, represent oxygen or a radical -NZ-;
- the radicals Z, which are identical or different, denote hydrogen or a linear or branched C₁-C₁₈ alkyl radical, a C₅-C₁₂ cycloalkyl radical which may be substituted with one or more C₁-C₄ alkyl radicals;
- T₃, T₄ and T₅, which are identical or different, are chosen from hydrogen; an alkali metal; an ammonium radical which is optionally substituted with one or more alkyl or hydroxyalkyl radicals; a linear or branched C₁-C₁₈ alkyl radical; a C₅-C₁₂ cycloalkyl radical which is optionally substituted with one or more C₁-C₄ alkyl radicals; a polyoxyethylenated radical comprising from 1 to 6 ethylene oxide units and whose terminal OH group is methylated; a radical of the following formula (3), (4) or (5): 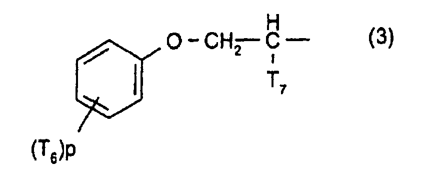 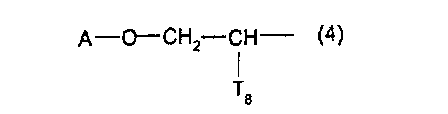 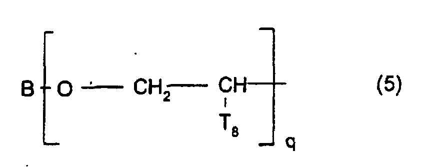
in which:
- T₆ is hydrogen or a methyl radical;
- T₇ is a C₁-C₉ alkyl radical;
- p is an integer ranging from 0 to 3;
- q is an integer ranging from 1 to 10;
- A is a C₄-C₈ alkyl radical or a C₅-C₈ cycloalkyl radical;
- B is chosen from a linear or branched C₁-C₈ alkyl radical; a C₅-C₈ cycloalkyl radical; an aryl radical which is optionally substituted with one or more C₁-C₄ alkyl radicals;
- T₈ is hydrogen or a methyl radical.

18. Emulsion according to Claim 17, where the UV-screening agent of the triazine type corresponds to the following formula: 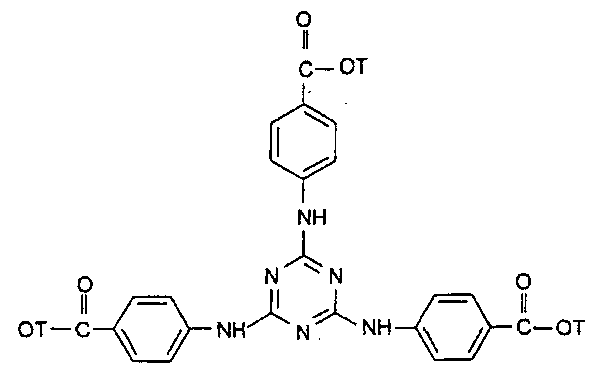 in which T denotes a 2-ethylhexyl radical.

19. Emulsion according to Claim 17, where the UV-screening agent of the triazine type corresponds to the following formula: 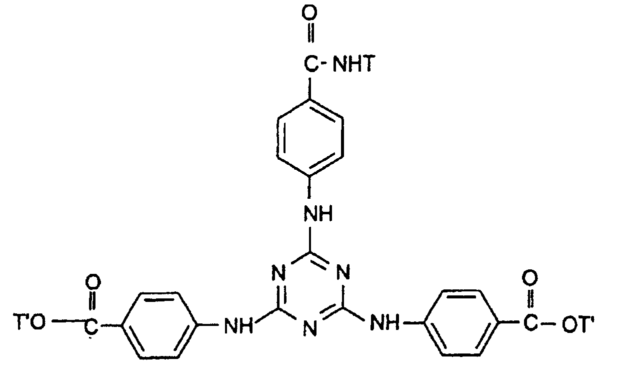 in which T' denotes a 2-ethylhexyl radical and T denotes a tert-butyl radical.

20. Emulsion according to Claim 15, where the UV-screening agents of the triazine type are chosen from the insoluble derivatives of s-triazine carrying benzalmalonate and/or phenylcyanoacrylate groups.

21. Emulsion according to Claim 20, where the UV-screening agents of the triazine type are chosen from the following compounds:
- 2,4,6-tris(diethyl 4'-aminobenzalmalonate)-s-triazine,
- 2,4,6-tris(diisopropyl 4'-aminobenzalmalonate)-s-triazine,
- 2,4,6-tris(dimethyl 4'-aminobenzalmalonate)-s-triazine,
- 2,4,6-tris(ethyl α-cyano-4-aminocinnamate)-s-triazine.

22. Emulsion according to Claim 15, where the UV-screening agents of the triazine type are chosen from those corresponding to the following formula: 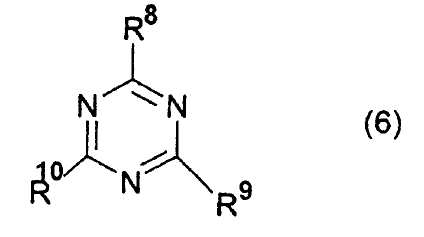 in which R⁸, R⁹, R¹⁰ are independently phenyl, phenoxy, pyrrolo, in which the phenyl, phenoxy and pyrrolo are optionally substituted with one, two or three substituents chosen from OH, C₁-C₁₈ alkyl or alkoxy, C₁-C₁₈ carboxyalkyl, C₅-C₈ cycloalkyl, a methylidenecamphor group, a group -(CH=CH)ₙ(CO)-OR⁴, with R⁴ either C₁-C₁₈ alkyl or cinnamyl, and n is equal to 0 or 1.

23. Emulsion according to Claim 15, where the UV-screening agents of the triazine type are chosen from the insoluble derivatives of s-triazine carrying benzotriazole and/or benzothiazole groups.

24. Emulsion according to Claim 23, where the insoluble UV-screening agents of the triazine type are chosen from:
- 2,4, 6-tris[(3'-benzotriazol-2-yl-2'-hydroxy-5'-methyl)phenylamino]-s-triazine,
- 2,4,6-tris[(3'-benzotriazol-2-yl-2'-hydroxy-5'-ter-octyl)phenylamino]-s-triazine.

25. Emulsion according to Claim 15, where the organic UV-screening agents of the triazole type correspond to the following formula (7): 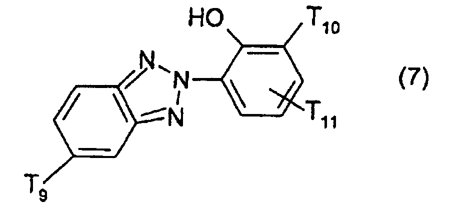 in which T₉ denotes a hydrogen atom or a C₁-C₁₈ alkyl radical; T₁₀ and T₁₁, which are identical or different, denote a C₁-C₁₈ alkyl radical which is optionally substituted with a phenyl.

26. Emulsion according to Claim 25, where the compound of formula (7) is chosen from the following compounds:

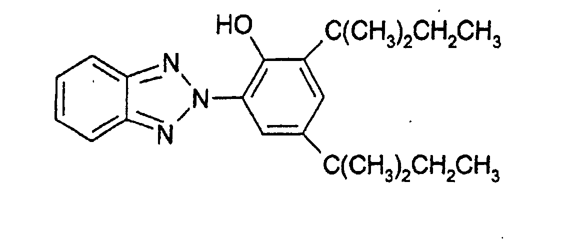

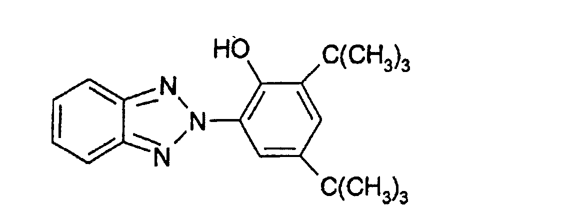

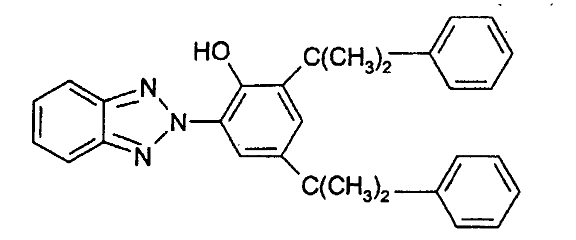

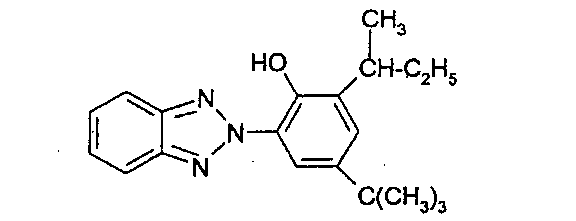

27. Emulsion according to Claim 15, where the insoluble UV-screening agent in micronized form is [2,4'-dihydroxy-3-(2H-benzotriazol-2-yl)-5-(1,1,3,3-tetramethylbutyl)-2'-n-octoxy-5'-benzoyl]diphenylmethane having the structure:

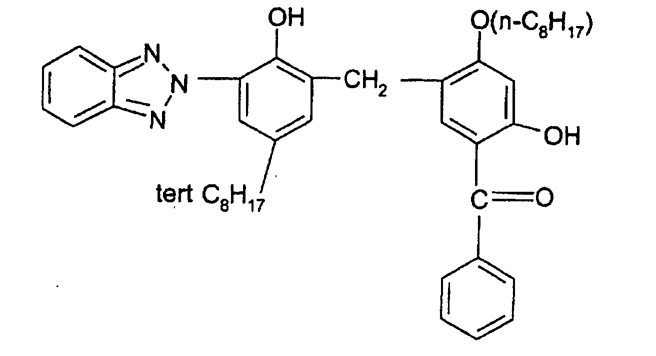

28. Emulsion according to Claim 15, where the organic UV-screening agents of the triazole type are chosen from the methylenebis(hydroxyphenylbenzotriazole) derivatives having the following structure: 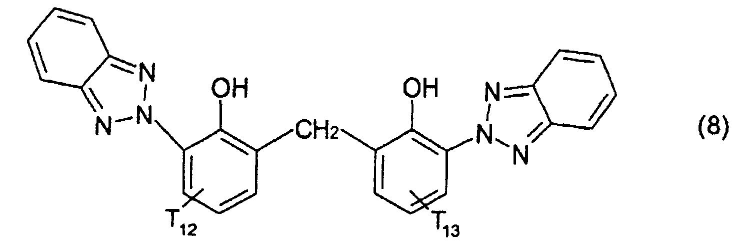 in which the radicals T₁₂ and T₁₃, which are identical or different, denote a C₁-C₁₈ alkyl radical which may be substituted with one or more radicals chosen from a C₁-C₄ alkyl, a C₅-C₁₂ cycloalkyl or an aryl residue.

29. Emulsion according to Claim 28, where the compound of formula (8) is chosen from the group consisting of the compounds having the following structure:

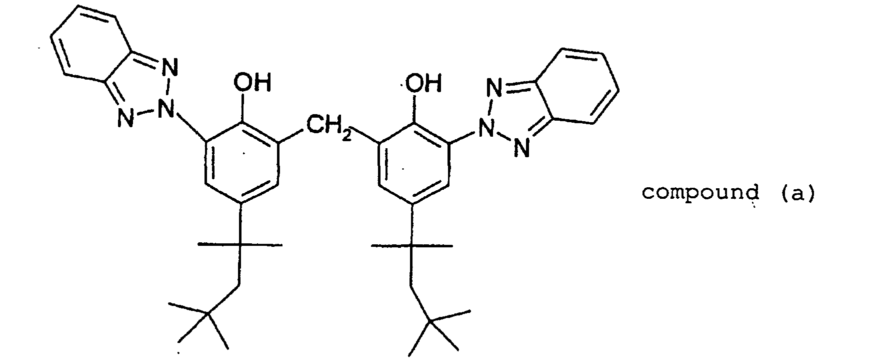

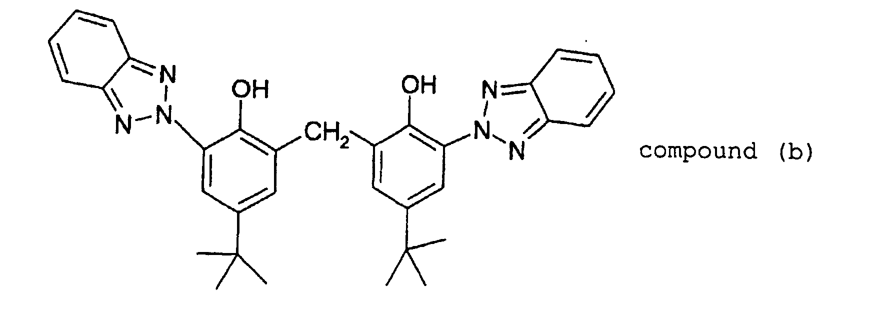

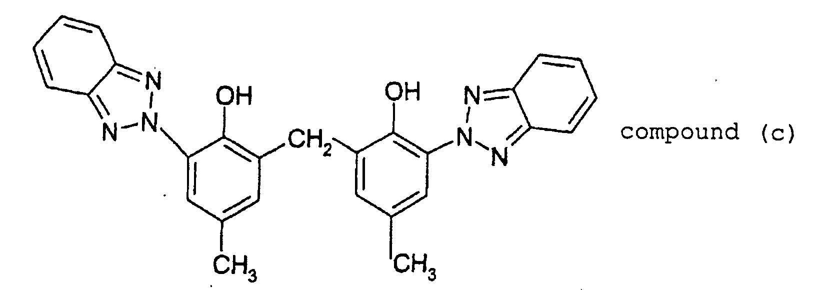

30. Emulsion according to Claim 15, where the organic screening agents of the vinylamide type correspond to the following formula:
T₁₄-(Y)r-C(=O)-C(T₁₅)=C(T₁₆)-N(T₁₇) (T₁₈) (9)
in which T₁₄ is a C₁-C₁₈, preferably C₁-C₅, alkyl radical or a phenyl group which is optionally substituted with one, two or three radicals chosen from OH, C₁-C₁₈ alkyl, C₁-C₈ alkoxy, or a group -C(=O)-OT₁₉ where T₁₉ is a C₁-C₁₈ alkyl; T₁₅, T₁₆, T₁₇ and T₁₈, which are identical or different, denote a C₁-C₁₈, preferably C₁-C₅, alkyl radical or a hydrogen atom; Y is N or O and r is equal to 0 or 1.

31. Emulsion according to Claim 30, where the compounds of formula (9) are chosen from:
- 4-octylamino-3-penten-2-one;
- ethyl 3-octylamino-2-butenoate;
- 3-octylamino-1-phenyl-2-buten-1-one;
- 3-dodecylamino-1-phenyl-2-buten-1-one.

32. Emulsion according to Claim 15, where the organic screening agents of the cinnamamide type correspond to the following formula: 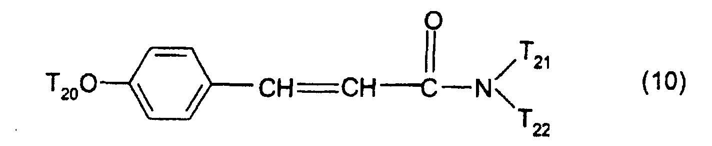 in which OT₂₀ is a hydroxyl or C₁-C₄ alkoxy, preferably methoxy or ethoxy, radical; T₂₁ is hydrogen, C₁-C₄ alkyl, preferably methyl or ethyl; T₂₂ is a group -(CONH)s-phenyl where s is equal to 0 or 1 and the phenyl group may be substituted with one, two or three groups chosen from OR, C₁-C₁₈ alkyl, C₁-C₈ alkoxy, or a group -C(=O)-OT₂₃ where T₂₃ is a C₁-C₁₈ alkyl and more preferably T₂₃ is a phenyl, 4-methoxyphenyl or phenylaminocarbonyl group.

33. Emulsion according to Claim 15, where the insoluble UV-screening agent is a cinnamamide dimer.

34. Emulsion according to Claim 33, where the insoluble UV-screening agent is the compound having the structure:

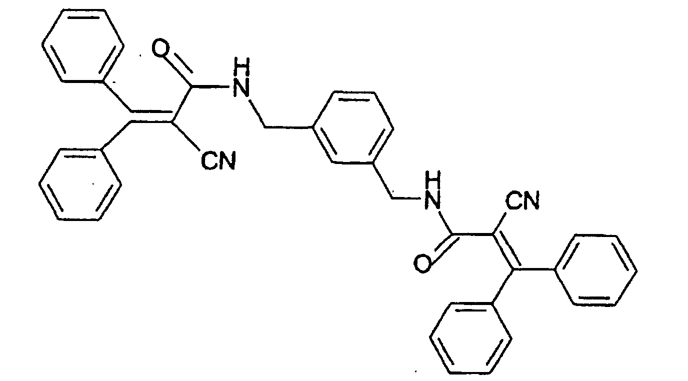

35. Emulsion according to any one of Claims 1 to 14, where the insoluble UV-screening agents are polyvalent metal salts of sulphonic or carboxylic organic UV-screening agents.

36. Emulsion according to any one of Claims 1 to 14, where the insoluble UV-screening agents are chosen from the polyvalent metal salts of sulphonated derivatives of benzylidenecamphor; the polyvalent metal salts of sulphonated derivatives of benzimidazole; the polyvalent metal salts of derivatives of cinnamic acid.

37. Emulsion according to any one of Claims 1 to 14, where the insoluble UV-screening agents are complexes of polyvalent metals or of ammonium or of ammonium substituted with organic UV-A and/or UV-B screening agents.

38. Emulsion according to Claim 37, where the insoluble UV-screening agent(s) in micronized insoluble form has a mean particle size ranging from 0.02 to 1.5 µm.

39. Emulsion according to Claim 38, where the insoluble UV-screening agent(s) in micronized insoluble form has a mean particle size ranging from 0.03 to 1.0 µm.

40. Emulsion according to any one of Claims 1 to 39, **characterized in that** the insoluble UV-screening agents) in micronized form can be obtained by a method of grinding the insoluble organic screening agent in the form of particles of coarse size in the presence of a surfactant.

41. Emulsion according to Claim 40, where the surfactant is chosen from alkyl polyglucosides having the structure CₙH₂ₙ₊₁O (C₆H₁₀O₅)ₓH in which n is an integer from 8 to 16 and x is the average degree of polymerization of the unit (C₆H₁₀O₅) and varies from 1.4 to 1.6.

42. Emulsion according to either of Claims 40 and 41, **characterized in that** the surfactant is used at a concentration ranging from 1 to 50% by weight relative to the insoluble screening agent in its micronized form.

43. Cosmetic or dermatological composition intended for the photoprotection of the skin and/or of the hair, **characterized in that** it comprises in a cosmetically acceptable carrier an emulsion as defined in any one of Claims 1 to 42.

44. Composition according to Claim 43, **characterized in that** it comprises, in addition, one or more additional organic screening agents active in UV-A and/or UV-B, soluble in at least one of the phases of the composition.

45. Composition according to Claim 44, **characterized in that** the said additional organic screening agents are chosen from the cinnamic derivatives; the salicylic derivatives; the camphor derivatives; the triazine derivatives; the dibenzoylmethane derivatives; the benzophenone derivatives; the dimers derived from α-alkylstyrene; the β,β'-diphenyl acrylate derivatives; the benzimidazole derivatives; the bisbenzoazolyl derivatives; the p-aminobenzoic acid derivatives; the polymer screening agents and silicone screening agents.

46. Composition according to any one of Claims 43 to 45, **characterized in that** it comprises, in addition, pigments or nanopigments of metal oxides, coated or otherwise.

47. Composition according to Claim 46, **characterized in that** the said pigments or nanopigments are chosen from titanium, zinc, iron, zirconium or cerium oxides and mixtures thereof, coated or otherwise.

48. Composition according to any one of Claims 43 to 47, **characterized in that** it comprises, in addition, at least one agent for tanning and/or for artificial tanning of the skin.

49. Composition according to any one of Claims 43 to 48, **characterized in that** it comprises, in addition, at least one adjuvant chosen from fatty substances, organic solvents, thickeners, demulcents, opacifiers, stabilizers, emollients, antifoaming agents, moisturizing agents, perfumes, preservatives, polymers, sequestrants, propellants, alkalinizing or acidifying agents.

50. Composition according to any one of Claims 43 to 49, **characterized in that** it is a composition for protecting the human epidermis 'or an anti-sun composition and **in that** it is provided in the form of a nonionic vesicular dispersion, a cream, a milk, a gel, a gel cream, a suspension, a dispersion, a powder, a solid stick, a foam or a spray.

51. Composition according to any one of Claims 43 to 49, **characterized in that** it is a make-up composition for the eyelashes, the eyebrows or the skin and **in that** it is provided in solid or pasty, anhydrous or aqueous form, in the form of an emulsion, a suspension or a dispersion.

52. Composition according to any one of Claims 43 to 49, **characterized in that** it is a composition intended for protecting the hair against ultraviolet rays and **in that** it is provided in the form of a shampoo, a lotion, a gel, an emulsion, a nonionic vesicular dispersion.

53. Use of the emulsion defined in any one of Claims 1 to 42 for the manufacture of cosmetic compositions for protecting the skin and/or the hair against ultraviolet radiation, in particular solar radiation.
